(19) Europäisches Patentamt
European Patent Office
Office européen des brevets

(11) EP 3 124 058 A1

(12) EUROPEAN PATENT APPLICATION
published in accordance with Art. 153(4) EPC

(43) Date of publication:
**01.02.2017 Bulletin 2017/05**

(21) Application number: **14887535.4**

(22) Date of filing: **06.08.2014**

(51) Int Cl.:
***A61L 31/00*** (2006.01) ***C07K 9/00*** (2006.01)

(86) International application number:
**PCT/JP2014/070703**

(87) International publication number:
**WO 2015/145797 (01.10.2015 Gazette 2015/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.03.2014 JP 2014068333**

(71) Applicant: **Otsuka Chemical Co., Ltd.**
**Osaka-shi,**
**Osaka 540-0021 (JP)**

(72) Inventors:
- **SAIJO, Hayato**
  **Kyoto-shi**
  **Kyoto 600-8813 (JP)**
- **OCHIAI, Hirofumi**
  **Kyoto-shi**
  **Kyoto 600-8813 (JP)**
- **SHIMODA, Taiji**
  **Kyoto-shi**
  **Kyoto 600-8813 (JP)**

(74) Representative: **Weickmann & Weickmann PartmbB**
**Postfach 860 820**
**81635 München (DE)**

# (54) HEMOSTATIC PHARMACEUTICAL COMPOSITION

(57) The object of the present invention is to provide a hemostatic pharmaceutical composition that has higher usability compared to a hemostatic agent employing conventional biogels and may form a transparent and homogeneous hydrogel in a broad pH.

The present invention provides a hemostatic pharmaceutical composition comprising a sugar chain-polypeptide complex characterized in that said polypeptide in said sugar chain-polypeptide complex is a polypeptide comprising an amino acid sequence in which polar and nonpolar amino acid residues are alternately arranged, and one or more sugar chains are bound to said polypeptide.

EP 3 124 058 A1

## Description

### Technical Field

The present invention relates to a hemostatic pharmaceutical composition comprising a sugar chain-polypeptide complex.

### Background Art

Biogels such as hydrogel and fibrin glue are utilized as research matrix for three dimensional culture etc., surgical matrix such as peri/post-operation hemostatics or wound healing sheets, drug delivery system (DDS), and the like.
However, since many of these employ materials of biological origin, risks such as infection from microorganisms such as viruses, immunogenicity, and transmission of diseases exist with use thereof. For example, although fibrin glue has high utility value as a hemostatic during surgery, because the source material is derived from human blood, there had been multiple cases upon actual use during surgery of patients being infected by hepatitis virus that had contaminated the fibrin glue, thus causing a major social problem. There is also a problem that gels of homogeneous quality cannot always be supplied with biogels of biological origin.
In contrast to biogels of biological origin, biogels manufactured by chemical synthesis are known to have no risk of infection and be capable of providing gels of homogeneous quality (Patent Literature 1). However, biogels known to date require procedures such as buffer exchange or substitution and mixing of multiple agents when forming a gel, and operation is complicated. Moreover, not only reagents or solvents to be used in combination are limited because solubility is low depending on the pH range, but there are also problems such as limitation of applicable sites (affected sites) and clogging of syringes or tubes upon use. In addition, low solubility (i.e. not transparent), particularly in the neutral range which is close to the biological pH, will complicate employment in situations that require visibility such as the surgery field.

### Citation List

[Patent Literature 1] U.S. Patent No. 5670483

### Summary of the Invention

### Problem to be Solved by the Invention

The object of the present invention is to provide a hemostatic pharmaceutical composition that has higher usability compared to a hemostatic agent employing conventional biogels and may form a transparent and homogeneous hydrogel in a broad pH.

### Means for Solving the Problem

As a result of extensive investigation by the present inventors to solve said problem, not only was it surprisingly found that a sugar chain-polypeptide complex manufactured by binding a sugar chain to a polypeptide comprising an amino acid sequence in which polar and nonpolar amino acid residues are alternately arranged shows high water-solubility and forms a transparent and homogeneous hydrogel in a broad pH range, particularly in the neutral range, but it was also found that this hydrogel is extremely useful as a hemostatic agent, thus leading to the completion of the present invention.
In other words, the present invention provides a hemostatic pharmaceutical composition comprising a sugar chain-polypeptide complex characterized in that said polypeptide in said sugar chain-polypeptide complex is a polypeptide comprising an amino acid sequence in which polar and nonpolar amino acid residues are alternately arranged, and one or more sugar chains are bound to said polypeptide.
Moreover, one embodiment of the present invention is characterized in that said polypeptide in said sugar chain-polypeptide complex is a polypeptide comprising an amino acid sequence consisting of 8 - 34 amino acid residues in which polar and nonpolar amino acid residues are alternately arranged.
Moreover, one embodiment of the present invention is characterized in that said sugar chain-polypeptide complex may form a hydrogel comprising a P sheet structure by self-assembly in an aqueous solution having a pH around neutral.
Moreover, one embodiment of the present invention is characterized in that the concentration of said sugar chain-polypeptide complex contained in said hemostatic pharmaceutical composition is 0.1% by weight - 20% by weight.

Moreover, one embodiment of the present invention is characterized in that the total number of sugar residues present in the one or more sugar chains bound to said polypeptide is 5 or more.

Moreover, one embodiment of the present invention is characterized in that the number of sugar chains bound to said polypeptide is 1, 2, or 3.

Moreover, one embodiment of the present invention is characterized in that sugar chains are bound to every amino acid up to position x counting from the amino acid residue positioned at the N-terminal of said polypeptide and every amino acid up to position y counting from the amino acid residue positioned at the C-terminal (wherein x and y are integers, $x \geq 0$, $y \geq 0$, and $x + y$ is the total number of sugar chains bound to the polypeptide).

Moreover, one embodiment of the present invention is characterized in that said sugar chain is a sugar chain with a branch.

Moreover, one embodiment of the present invention is characterized in that said pharmaceutical composition is in a hydrogel state.

Those skilled in the art shall recognize that an invention of any combination of one or more characteristics of the present invention described above is also encompassed in the scope of the present invention.

Effects of the Invention

Because the hemostatic pharmaceutical composition according to the present invention has high water-solubility in a broad pH range comprising the neutral range and forms a uniform and transparent hydrogel, it is less subject to limitation from reagents or solvents that are used in combination, and can be employed as a hemostatic agent with high usability. Moreover, because it may be employed in a broad pH range, it is less subject to limitation of applicable sites (affected sites).

Moreover, because the sugar chain-polypeptide complex contained in the hemostatic pharmaceutical composition according to the present invention has high water-solubility in a broad pH range comprising the neutral range and forms a uniform and transparent hydrogel, sol and gel states may be reversibly present in a neutral pH. In other words, the sugar chain-polypeptide complex can form a gel state once and then be in a sol state by mechanical stirring, and still be in a gel state again. Accordingly, it can be distributed in a gel state (i.e. a Ready-to-Use state), and does not require complicated operations as with other peptidic gels such as buffer exchange (or substitution) in order to achieve a neutral pH after forming a gel at a pH suitable for gelling (e.g. acidic pH). In other words, the sugar chain-polypeptide complex according to the present invention is extremely superior in operativity compared to other peptidic gels. In addition, because the pH range that the hemostatic pharmaceutical composition according to the present invention can be used in is broad, problems such as clogging of syringes and tubes upon use occur less often.

Moreover, because the sugar chain-polypeptide complex contained in the hemostatic pharmaceutical composition according to the present invention is modified by a sugar chain that exists *in vivo* in animals, antigenicity is reduced compared to a peptide without any modification. In addition, the sugar chain-polypeptide complex contained in the hemostatic pharmaceutical composition according to the present invention has almost no risk of producing toxicity such as that seen with a compound modified with e.g. polyethylene glycol (PEG). Accordingly, the hemostatic pharmaceutical composition according to the present invention has high safety for biological use.

Moreover, because the hemostatic pharmaceutical composition according to the present invention forms a uniform and transparent hydrogel under physiologic conditions (neutral range) and has low antigenicity, it is preferable as a hydrogel for *in vivo* animal use.

Brief Description of the Drawings

Figure 1 shows the result of carrying out circular dichroism (CD) measurement for each composition of C(DiGlcNAc)-(RADA)4 dissolved in ultrapure water, saline, or phosphate buffer.

Figure 2 shows the result of carrying out circular dichroism (CD) measurement for each composition of (RADA)4 dissolved in ultrapure water, saline, or phosphate buffer.

Figure 3 is compares the ability of C(DiGlcNAc)-(RADA)4 and (RADA)4 to form a fibrous structure.

Figure 4 shows the storage elastic modulus in aqueous solution state of C(DiGlcNAc)-(RADA) 4 and (RADA)4.

Figure 5 shows the storage elastic modulus after addition of salt for C(DiGlcNAc)-(RADA)4 and (RADA)4.

Figure 6 shows the distribution of hemostatic effect score at 3 minutes after application when C(DiGlcNAc)-(RADA)4 or (RADA)4 is applied in a rat liver puncture model.

Description of Embodiments

The sugar chain-polypeptide complex contained in the hemostatic pharmaceutical composition according to the present invention may be of biological origin or may be manufactured by chemical synthesis, but it is preferably manufactured by chemical synthesis from aspects of stability of safety or quality and uniformity of sugar chains.

The sugar chain-polypeptide complex contained in the hemostatic pharmaceutical composition according to the present invention may e.g. self-assemble in an aqueous solution via interactions such as electrostatic interaction between peptide molecules, hydrogen bonding, and hydrophobic interaction. A sugar chain-polypeptide complex "self-assembles" in an aqueous solution as used herein means that polypeptides spontaneously assemble with each other via some kind of interaction (e.g. electrostatic interaction, hydrogen bonding, van der Waals force, and hydrophobic interaction) in an aqueous solution, and is not to be construed as having a limiting meaning.

The sugar chain-polypeptide complex contained in the hemostatic pharmaceutical composition according to the present invention may self-assemble and form a P sheet structure in an aqueous solution. Further, a hydrogel may be formed by multiple layering of said P sheet structures. The method for confirming that the sugar chain-polypeptide complex forms a P sheet structure in an aqueous solution is not particularly limited, and it can be verified by e.g. measuring the circular dichroism (CD) of an aqueous solution comprising the sugar chain-polypeptide complex. Because generally as a characteristic of a molecule having P sheet structure maximum is seen at a wavelength around 197 nm and minimum is seen at a wavelength around 216 nm, P sheet structure formation can be confirmed by verifying peaks around these wavelengths by circular dichroism measurement.

Because the sugar chain-polypeptide complex contained in the hemostatic pharmaceutical composition according to the present invention comprises an amino acid sequence in which polar and nonpolar amino acid residues are alternately arranged, only polar amino acid residues may be arranged on one side of the P sheet structure and only nonpolar amino acid residues may be arranged on the other side when forming a P sheet structure in an aqueous solution. Accordingly, said P sheet structure may assemble in such a way to hide the hydrophobic sides (the sides with only nonpolar amino acid residues arranged) to form a bilayered structure. Subsequently, this P sheet layered structure will be extended as molecular self-assembly progresses and thus may form a three dimensional conformation (e.g. a hydrogel).

A "pH around neutral" as used herein means that the pH is around 7.0, more specifically that the pH is in the range of 5.0 - 9.0, preferably that the pH is in the range of 6.0 - 8.0.

The concentration of the sugar chain-polypeptide complex contained in the hemostatic pharmaceutical composition of the present invention can be appropriately adjusted by those skilled in the art according to the applicable symptoms or applicable sites, and for example may be 0.1% by weight - 20% by weight, preferably 0.2% by weight - 15% by weight, and further preferably 0.5% by weight - 10% by weight. When the concentration of the sugar chain-polypeptide complex contained in the hemostatic pharmaceutical composition is 0.1% by weight or less, there is a possibility that a hydrogel will not be properly formed. Note that a hydrogel herein broadly means a gel or sol composition in which the dispersion medium is substantially water, and shall not be construed as having a limited meaning.

The hemostatic pharmaceutical composition of the present invention may be employed in an aqueous solution state or in a hydrogel state. The method for preparing said aqueous solution or said hydrogel is not limited. For example, a hemostatic pharmaceutical composition in aqueous solution state may be obtained by dissolving a sugar chain-polypeptide complex in ultrapure water, or a hemostatic pharmaceutical composition in hydrogel state may be obtained by further adding a solvent (such as physiological saline, PBS, etc.) comprising a salt to the aqueous solution of a sugar chain-polypeptide complex dissolved in ultrapure water.

Moreover, a hemostatic pharmaceutical composition is not limited to those comprising only a sugar chain-polypeptide complex and a solvent (water, PBS, physiological saline, etc.), and may comprise various other components. For example, by containing an agent having a disinfecting/sterilizing component, not only hemorrhage from the wound could be stopped but sterilization/disinfection of the wound can also be carried out at the same time.

As shown in the Examples of the present specification, the hemostatic pharmaceutical composition of the present invention has low storage elastic modulus in aqueous solution state and high storage elastic modulus in hydrogel state. Moreover, the hemostatic pharmaceutical composition of the present invention forms a clear hydrogel having uniform fibrous structure in a broad pH range including the neutral range. The applicable symptoms or applicable sites of the hemostatic pharmaceutical composition of the present invention are not particularly limited, and by virtue of the above nature, can be preferably employed particularly for hemostasis when visual confirmation of the state of affected site is necessary such as during surgery, hemostasis of a hemorrhage site that is a blind spot, hemostasis of a broad hemorrhage site, hemostasis of hemorrhage site of stereoscopic or complicated form, and the like.

One embodiment of the present invention is characterized in that the sugar chain-polypeptide complex contained in the hemostatic pharmaceutical composition may self-assemble in an aqueous solution having a pH around neutral to form a hydrogel comprising a P sheet structure. Those that may self-assemble even in an aqueous solution having a pH other than around neutral to form a hydrogel comprising a P sheet structure are not excluded, as long as they possess

the said characteristic.

The sugar chain-polypeptide complex contained in the hemostatic pharmaceutical composition according to the present invention comprises a polypeptide comprising an amino acid sequence in which polar and nonpolar amino acid residues are alternately arranged. The length of said amino acid sequence is not limited, and preferably it may be an amino acid sequence consisting of 8 - 34 amino acid residues, more preferably an amino acid sequence consisting of 12 - 25 amino acid residues, and further preferably an amino acid sequence consisting of 16 - 21 amino acid residues.

The sugar chain-polypeptide complex contained in the hemostatic pharmaceutical composition according to the present invention comprises a polypeptide comprising an amino acid sequence in which polar and nonpolar amino acid residues are alternately arranged. An "amino acid" as used herein is employed in its broadest meaning, and comprises not only protein-constituting amino acids but also non-protein-constituting amino acids such as amino acid variants and derivatives. Those skilled in the art shall recognize in light of this broad definition that examples of an amino acid herein include protein-constituting L-amino acids; D-amino acids; chemically modified amino acids such as amino acid variants and derivatives; non-protein-constituting amino acids such as norleucine, (β-alanine, and ornithine; as well as chemically synthesized compounds having properties well-known in the art that are characteristics of amino acids. Examples of a non-protein-constituting amino acid include α-methylamino acids (such as a-methylalanine), D-amino acids, histidine-like amino acids (such as 2-amino-histidine, (β-hydroxy-histidine, homohistidine, α-fluoromethyl-histidine, and α-methyl-histidine), amino acids having excess methylene on the side chain ("homo" amino acids), and amino acids having the carboxylate functional group amino acid in the side chain substituted by a sulfonate group (such as cysteic acid). In a preferred aspect of the present invention, the amino acids employed herein may be protein-constituting amino acids.

A polar amino acid residue as used herein is not particularly limited as long as it is an amino acid residue of which the side chain may have polarity, examples of which include acidic amino acid residues and basic amino acid residues. Examples of an acidic amino acid residue as used herein include an aspartic acid (Asp:D) residue and glutamic acid (Glu:E), and examples of a basic amino acid include arginine (Arg:R), lysine (Lys:K), and histidine (His:H).

Note that for example representations such as "aspartic acid (Asp:D)" as used herein means that a three-letter representation "Asp" and one-letter representation "D" may be employed as abbreviations of aspartic acid.

Moreover, in the present specification, among neutral amino acid residues, amino acid residues comprising a hydroxyl group, an acid amide group, a thiol group, and the like are included in polar amino acid residues as those having polarity. For example, in the present specification, tyrosine (Tyr:Y), serine (Ser:S), threonine (Thr:T), asparagine (Asn:N), glutamine (Gln:Q), and cysteine (Cys:C) are included in polar amino acid residues.

A nonpolar amino acid residue as used herein is not particularly limited as long as it is an amino acid of which the side chain does not have polarity, examples of which include alanine (Ala:A), valine (Val:V), leucine (Leu:L), isoleucine (Ile:I), methionine (Met:M), phenylalanine (Phe:F), tryptophan (Trp:W), glycine (Gly:G), and proline (Pro:P).

In the sugar chain-polypeptide complex contained in the hemostatic pharmaceutical composition according to the present invention, "an amino acid sequence in which polar and nonpolar amino acid residues are alternately arranged" is preferably those where said amino acid sequence may be a repetitive sequence "RADA" (2 - 8 repeats, preferably 3 -6 repeats), and more preferably where said amino acid sequence may be RADARADARADARADA (SEQ ID NO. 1) or RADARADARADARADARADA (SEQ ID NO. 2).

A "sugar chain" as used herein refers to a compound composed of a string of one or more unit sugars (monosaccharide and/or derivatives thereof) . When it is a string of two unit sugars, each unit sugar is bound to each other by a dehydration condensation by a glycoside bond. Examples of such a sugar chain include, are but not limited to, a broad range such as monosaccharides and polysaccharides contained *in vivo* (glucose, galactose, mannose, fucose, xylose, N-acetylglucosamine, N-acetylgalactosamine, sialic acid, and complexes and derivatives thereof), as well as sugar chains that were degradated or induced from complex biomolecules such as degradated polysaccharides, glycoproteins, proteoglycans, glycosaminoglycans, and glycolipids. The sugar chain may be linear or branched chain.

A "sugar chain" as used herein also includes sugar chain derivatives. Examples of a sugar chain derivative include, but are not limited to, sugar chains wherein the sugar configuring the sugar chain is for example a sugar possessing a carboxy group (e.g. aldonic acid in which C-1 position is oxidized to become a carboxylic acid (such as D-gluconic acid which is oxidized D-glucose) and uronic acid in which the terminal C atom became a carboxylic acid (D-glucuronic acid which is oxidized D-glucose)), a sugar possessing an amino group or an amino group derivative (such as D-glucosamine and D-galactosamine), a sugar possessing both amino and carboxy groups (such as N-glycoylneuraminic acid and N-acetylmuramic acid), a deoxylated sugar (such as 2-deoxy-D-ribose), a sulfated sugar comprising a sulfate group, and a phosphorylated sugar comprising a phosphate group.

In the sugar chain-polypeptide complex according to the present invention, the sugar chain to be bound to the polypeptide is not particularly limited, but is preferably a sugar chain that exists *in vivo* as a glycoconjugate (such as a glycopeptide (or a glycoprotein), a proteoglycan, and a glycolipid) with respect to biocompatibility. Such sugar chains include N-linked sugar chains, O-linked sugar chain, and the like, which are sugar chains that are bound *in vivo* to peptides (or proteins) as glycopeptides (or glycoproteins).

In the sugar chain-polypeptide complex according to the present invention, for example a disialo sugar chain,

an asialo sugar chain, a diGlcNAc sugar chain, a dimannose (DiMan) sugar chain, a GlcNAc sugar chain, a maltotriose sugar chain, a maltose sugar chain, a maltotetraose sugar chain, a maltoheptaose sugar chain, a β-cyclodextrin sugar chain, and a γ-cyclodextrin sugar chain can be employed for the sugar chain to be bound to the polypeptide.

More specifically, the sugar chain employed in the present invention may be a disialo sugar chain shown by the following Formula (1), an asialo sugar chain shown by the following Formula (2), a diGlcNAc sugar chain shown by the following Formula (3), a dimannose sugar chain shown by the following Formula (4), a GlcNAc sugar chain shown by the following Formula (5), a maltotriose sugar chain shown by the following Formula (6), a maltose sugar chain shown by the following Formula (7), a maltotetraose sugar chain shown by the following Formula (8), a maltoheptaose sugar chain shown by the following Formula (9), a β-cyclodextrin sugar chain shown by the following Formula (10), or a γ-cyclodextrin sugar chain shown by the following Formula (11).

[Chemical Formula 1]

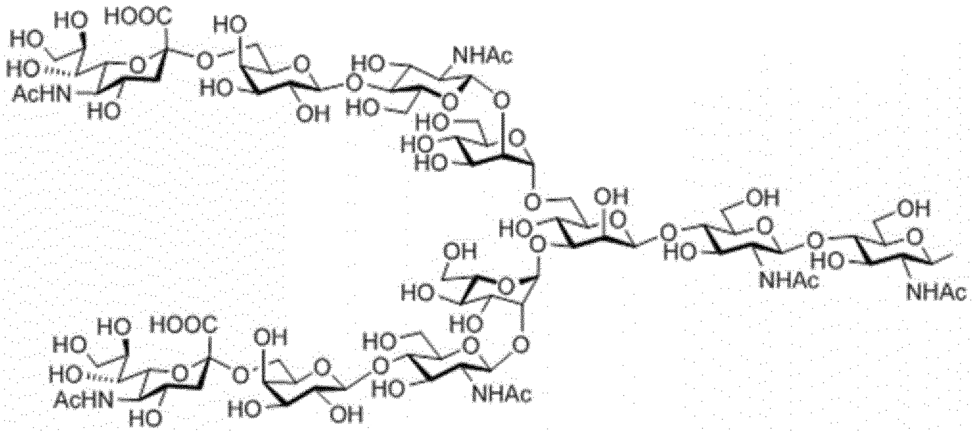

Formula (1) Disialo sugar chain

[Chemical Formula 2]

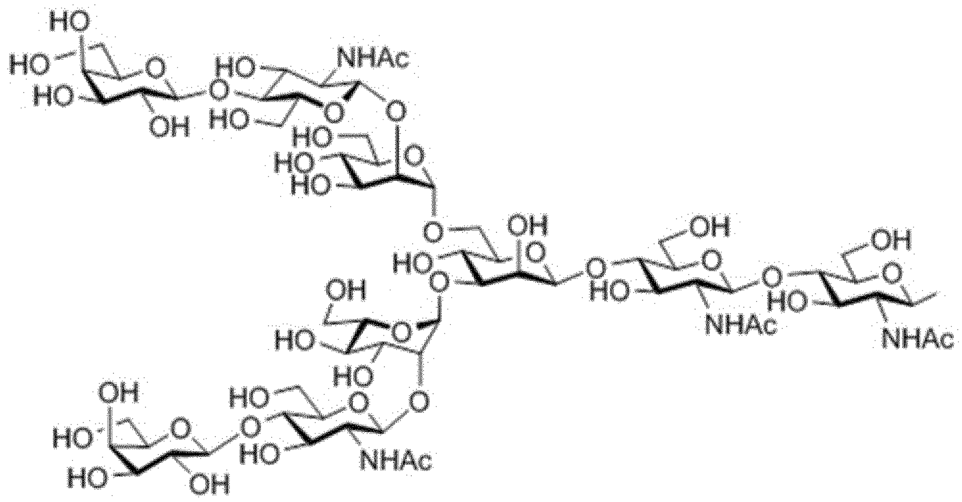

Formula (2) Asialo sugar chain

[Chemical Formula 3]

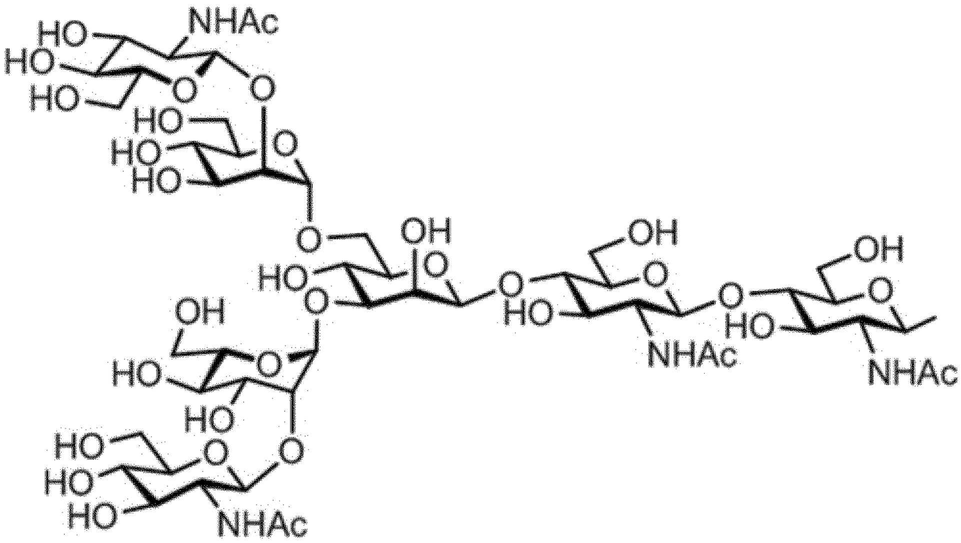

Formula (3) DiGlcNAc sugar chain

[Chemical Formula 4]

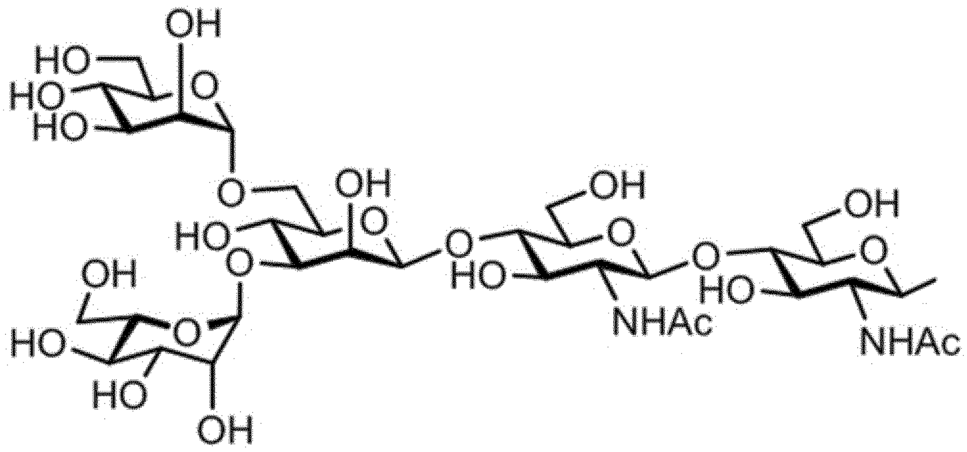

Formula (4) Dimannose sugar chain

[Chemical Formula 5]

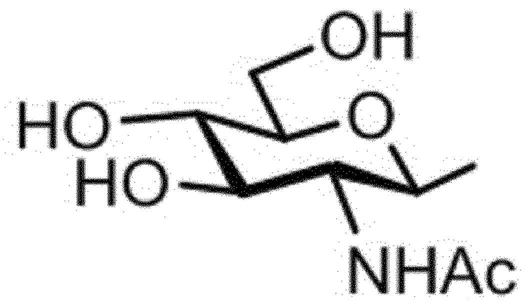

Formula (5) GlcNAc sugar chain

[Chemical Formula 6]

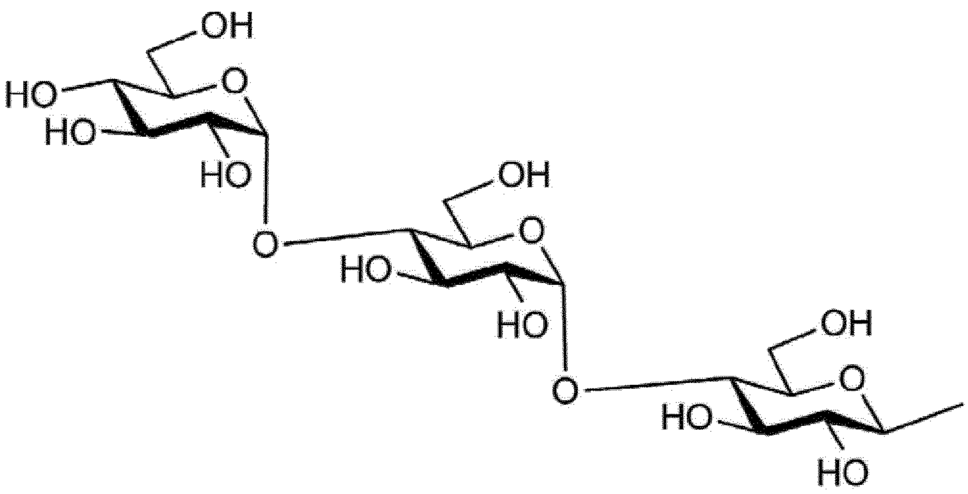

Formula (6) Maltotriose sugar chain

[Chemical Formula 7]

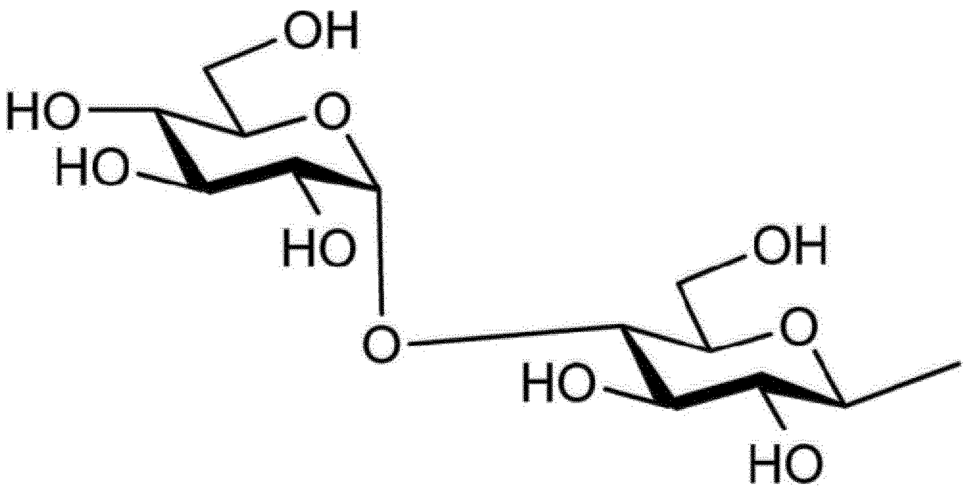

Formula (7) Maltose sugar chain

[Chemical Formula 8]

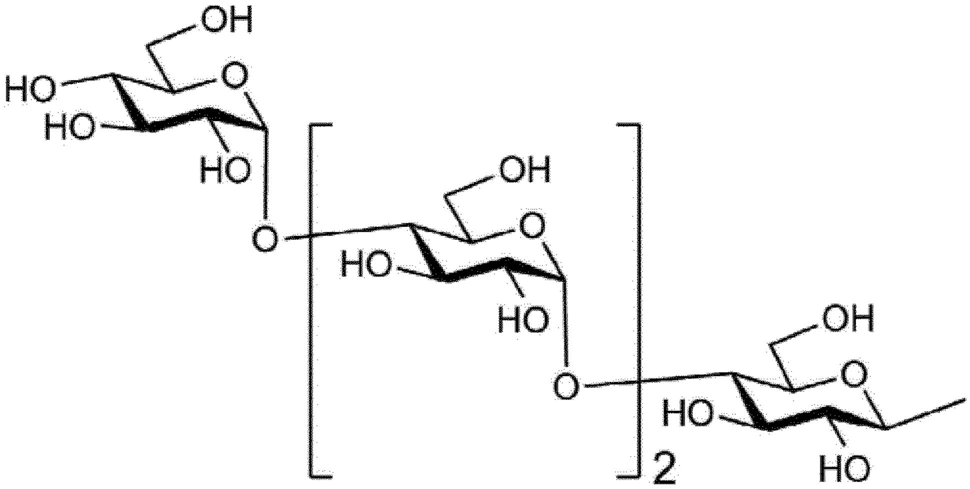

Formula (8) Maltotetraose sugar chain

[Chemical Formula 9]

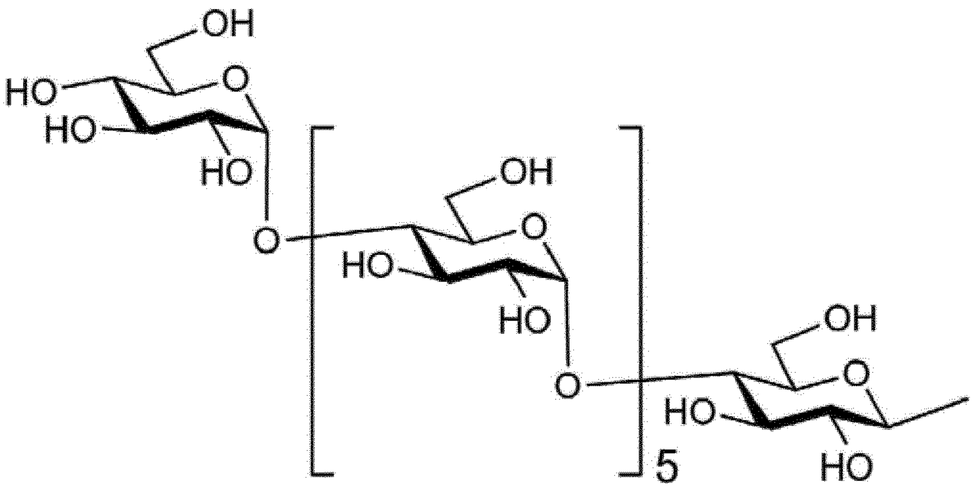

Formula (9) Maltoheptaose sugar chain

[Chemical Formula 10]

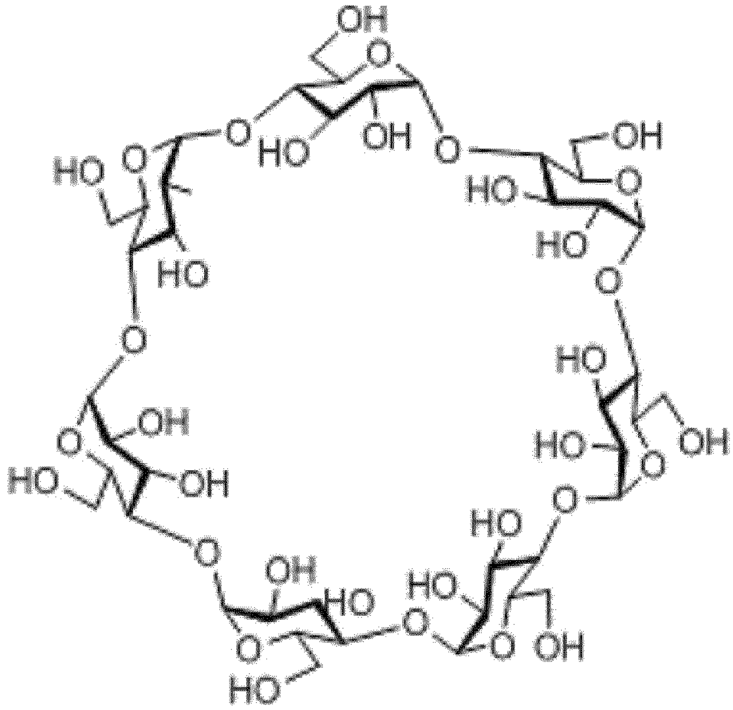

Formula (10) β-cyclodextrin sugar chain

[Chemical Formula 11]

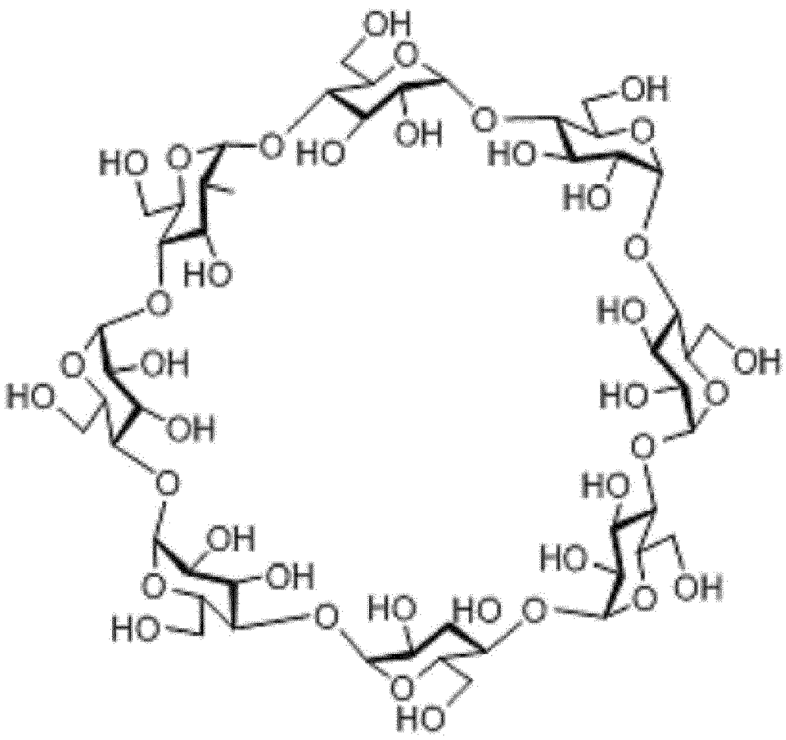

Formula (11) γ-cyclodextrin sugar chain

In the present invention, a sugar chain in which one or more sugars are lost from the non-reducing terminal of

the above disialo sugar chain, asialo sugar chain, diGlcNAc sugar chain, dimannose sugar chain, or maltoheptaose sugar chain can also be employed.

In the present invention, the amino acid residue to which a sugar chain is bound is not particularly limited. For example, a sugar chain can be bound to cysteine (Cys:C) or asparagine (Asn:N), preferably to cysteine (Cys:C).

In the present invention, the method for binding a sugar chain to an amino acid is not particularly limited. For example, a sugar chain may be directly bound to an amino acid residue, or a sugar chain may be bound to an amino acid residue via a linker.

Moreover, in the present invention, the amino acid residue to which a sugar chain is bound may be directly bound to "an amino acid sequence in which polar and nonpolar amino acid residues are alternately arranged," or may be bound via e.g. a linker.

Examples of such a linker can include an alkyl chain or a PEG chain possessing amino and carboxy groups at both ends so that it can form peptide bonds with an amino acid. Examples of such a linker can include - $NH-(CH_2)_n-CO-$ (wherein n is an integer and is not limited as long as it does not inhibit the linker function of interest, preferably an integer between 1 - 15) or $-NH-(CH_2CH_2O)_m-CH_2CH_2-CO-$ (wherein m is an integer and is not limited as long as it does not inhibit the linker function of interest, preferably an integer between 1 - 7), more specifically $-NH-(CH_2)_{11}-CO-$(C12 linker) or $-NH-(CH_2CH_2O)_3-CH_2CH_2-CO-$(PEG linker) and the like.

The sugar chain-polypeptide complex employed in the present invention can be manufactured by integrating a glycosylation step into a polypeptide synthesis method well-known to those skilled in the art. Although a method utilizing an enzyme represented by transglutaminase can be employed for glycosylation, there are problems in this case such as the need for a large amount of the sugar chain to be added, complication of purification after the final step, and limitations on glycosylation positions and sugar chains that can be added. As a result, although it is possible to employ this in a small scale synthesis such as for assays, it cannot be said to be a practical method for a large scale manufacturing.

As specific examples of an easy manufacturing method of the sugar chain-polypeptide complex employed in the present invention, a method for manufacturing a sugar chain-polypeptide complex by using Asn having a sugar chain bound thereto (glycosylated Asn) and applying a well-known peptide synthesis method such as solid and liquid phase synthesis (Method A), and a method for manufacturing a sugar chain-polypeptide complex by manufacturing a polypeptide in which an arbitrary amino acid residue is Cys according to a well-known peptide synthesis method, and then glycosylating the Cys by chemical synthesis (Method B) will be illustrated below. Those skilled in the art will be able to manufacture sugar chain-polypeptide complexes by various methods by referring to these manufacturing methods.

These Methods A and B can also be carried out in a combination of two or more. In case of a small scale synthesis employed for assays etc., the above method can further be used in combination with a sugar chain elongation reaction by a transferase. Method A is described in International Publication No. 2004/005330 (US2005222382 (A1)) and Method B is described in International Publication No. 2005/010053 (US2007060543 (A1)), the disclosures of which are incorporated herein by reference in their entireties. Moreover, manufacturing of sugar chains having uniform sugar chain structure employed in Methods A and B are described in e.g. International Publication No. 03/008431 (US2004181054 (A1)), International Publication No. 2004/058984 (US2006228784 (A1)), International Publication No. 2004/058824 (US2006009421 (A1)), International Publication No. 2004/070046 (US2006205039 (A1)), and International Publication No. 2007/011055, the disclosures of which are incorporated herein by reference in their entireties.

Method for manufacturing a sugar chain-polypeptide complex (Method A)

As outlined below, the sugar chain-polypeptide complex can be manufactured by e.g. solid phase synthesis employing Asn having a sugar chain bound thereto.

(1) The carboxy group of an amino acid having the amino group nitrogen protected with a lipophilic protecting group is bound to a resin. In this case, since the amino group nitrogen of the amino acid is protected with a lipophilic protecting group, self-condensation of amino acids with each other is prevented, and the resin and the amino acid react to form a bond.
(2) The lipophilic protecting group of the reactant obtained is detached to form a free amino group.
(3) This free amino group and the carboxy group of any amino acid having the amino group nitrogen protected with a lipophilic protecting group are subjected to an amidation reaction.
(4) The above lipophilic protecting group is detached to form a free amino group.
(5) The above steps (3) and (4) are repeated once or more times to yield a peptide of any number of any amino acids linked together, having a resin bound at one end and possessing a free amino group at the other end.
(6) When the free amino group of the peptide synthesized in above (5) is to be protected with an acetyl group, it is also preferred to acetylate with acetic anhydride, acetic acid, and the like.
(7) Finally, the resin is cleaved with an acid and a peptide having a desired amino acid sequence can be obtained.

Here, by employing a glycosylated Asn having the amino group nitrogen protected with a lipophilic protecting group instead of the amino acid having the amino group nitrogen protected with a lipophilic protecting group, and reacting the carboxy group of said asparagine moiety with the hydroxyl group of the resin in (1), a peptide possessing a glycosylated Asn at the C-terminal can be obtained.

Moreover, after (2), or after repeating (3) and (4) for any number of times that is once or more, by employing a glycosylated Asn having the amino group nitrogen protected with a lipophilic protecting group instead of the amino acid having the amino group nitrogen protected with a lipophilic protecting group in (3), a sugar chain can be bound at any position of the polypeptide.

In this way, by employing a glycosylated Asn having the amino group nitrogen protected with a lipophilic protecting group instead of the amino acid having the amino group nitrogen protected with a lipophilic protecting group two or more times in any of steps (1) and (3), sugar chains can be bound at any two or more positions of the polypeptide.

If, after binding the glycosylated Asn, the lipophilic protecting group is detached and a free amino group is formed, and step (7) is carried out immediately thereafter, a polypeptide possessing a glycosylated Asn at the N-terminal can be obtained.

A resin that provides the C-terminal as an amide group may be a resin ordinarily used in solid phase synthesis. For example, it is preferred to employ Rink-Amide-resin (from Merck & Co., Inc.), Rink-Amide-PEGA resin (from Merck & Co., Inc.), or NH-SAL-resin (from Watanabe Chemical Industries,Ltd.), which are functionalized with an amino group. Moreover, Fmoc-NH-SAL-resin-linker (from Watanabe Chemical Industries, Ltd.) and the like may be bound to Amino-PEGA-resin which is functionalized with an amino group (from Merck & Co., Inc.) and the like. The C-terminal amino acid of the peptide can be amidated by cleaving this resin and the peptide by an acid.

Moreover, examples of a resin which makes the C-terminal a carboxylic acid that can be employed are 2-chlorotrityl chloride resin functionalized with chlorine (from Merck & Co., Inc.), Amino-PEGA resin functionalized with an amino group (from Merck & Co., Inc.), NovaSyn TGT alcohol resin possessing a hydroxyl group (from Merck & Co., Inc.), Wang resin (from Merck & Co., Inc.), HMPA-PEGA resin (from Merck & Co., Inc.), and the like. Moreover, a linker may be present between Amino-PEGA resin and the amino acid, and examples of such a linker can include 4-hydroxymethylphenoxyacetic acid (HMPA), 4-(4-hydroxymethyl-3-methoxyphenoxy)-butylacetic acid (HMPB), and the like. H-Cys(Trt)-Trityl Nova PEG resin in which the C-terminal amino acid is bound to a resin in advance (from Merck & Co., Inc.) and the like can also be employed.

In regards to the binding between a resin and an amino acid having the amino group nitrogen protected with a lipophilic protecting group, for example in order to use a resin possessing a hydroxyl group or a resin functionalized with chlorine, the carboxy group of the amino acid is subjected to an ester binding with the resin. Moreover, when employing a resin functionalized with an amino group, the carboxy group of the amino acid is bound to the resin by an amide bond.

Note that 2-chlorotrityl chloride resin is preferred in that it can prevent racemization of terminal Cys when elongating the peptide chain in solid phase synthesis.

<u>Method for manufacturing a sugar chain-polypeptide complex - 2 (Method A)</u>

As outlined below, the sugar chain-polypeptide complex can be manufactured by e.g. liquid phase synthesis employing Asn having a sugar chain bound thereto.

(1) The carboxy group of an amino acid having the amino group nitrogen protected with a lipophilic protecting group is bound to an amino acid having the amino group free and the carboxy group protected or amidated.
(2) The lipophilic protecting group of the reactant obtained is detached to form a free amino group.
(3) This free amino group and the carboxy group of any amino acid having the amino group nitrogen protected with a lipophilic protecting group are subjected to an amidation reaction in solution. In this case, since the amino group nitrogen of the amino acid on the N-terminal side is protected with a lipophilic protecting group and the carboxy group on the C-terminal side is protected or amidated, self-condensation of amino acids with each other is prevented, and the free amino group and the carboxy group react to form a bond.
(4) The above lipophilic protecting group is detached to form a free amino group.
(5) The above steps (3) and (4) are repeated once or more times to yield a peptide of any number of any amino acids linked together, having the C-terminal carboxy group protected or amidated and possessing a free amino group at the N-terminal.
(6) When the free amino group of the peptide synthesized in above (5) is to be protected with an acetyl group, it is also preferred to acetylate with acetic anhydride, acetic acid, and the like.
(7) Finally, the side chain lipophilic protecting group is cleaved with an acid and a peptide having a desired amino acid sequence can be obtained.

Method for manufacturing a sugar chain-polypeptide complex - 3 (Method A)

As outlined below, the sugar chain-polypeptide complex can be manufactured by e.g. fragment synthesis method employing Asn having a sugar chain bound thereto.

(1) A polypeptide or a sugar chain-polypeptide complex having the amino group nitrogen protected with an acetyl group or a lipophilic protecting group is synthesized on a resin by (1)-(6) of the above method for manufacturing a sugar chain-polypeptide complex (Method A).
(2) The polypeptide or the sugar chain-polypeptide complex is cleaved from the resin under conditions that do not deprotect the side chain protecting group to obtain a polypeptide or a sugar chain-polypeptide complex possessing a free carboxy at the C-terminal and having the amino group nitrogen at N-terminal protected with an acetyl group or a lipophilic protecting group.
(3) The polypeptide or the sugar chain-polypeptide complex obtained having the amino group nitrogen protected with an acetyl group or a lipophilic protecting group is linked to a resin or a polypeptide by solid or liquid phase synthesis.
(4) The above lipophilic protecting group is detached to form a free amino group.
(5) The above steps (3) and (4) are repeated once or more times to yield a peptide of any number of any amino acids linked together.
(6) Finally, the resin is cleaved with an acid and a peptide having a desired amino acid sequence can be obtained.

Examples of a lipophilic protecting group can include a carbonate-or amide-based protecting group and the like such as a 9-fluorenylmethoxycarbonyl (Fmoc) group, a t-butyloxycarbonyl (Boc) group, a benzyl group, an allyl group, an allyloxycarbonyl group, and an acetyl group. In order to introduce a lipophilic protecting group to an amino acid, for example to introduce a Fmoc group, introduction can be carried out by adding 9-fluorenylmethyl-N-succinimidyl carbonate and sodium hydrogen carbonate and allowing to react. The reaction may be carried out at 0 - 50°C, preferably at room temperature for about 1 - 5 hours.
Those commercially available can also be used as the amino acid protected with a lipophilic protecting group, examples of which can include Fmoc-Ser-OH, Fmoc-Asn-OH, Fmoc-Val-OH, Fmoc-Leu-OH, Fmoc-Ile-OH, Fmoc-Ala-OH, Fmoc-Tyr-OH, Fmoc-Gly-OH, Fmoc-Lys-OH, Fmoc-Arg-OH, Fmoc-His-OH, Fmoc-Asp-OH, Fmoc-Glu-OH, Fmoc-Gln-OH, Fmoc-Thr-OH, Fmoc-Cys-OH, Fmoc-Met-OH, Fmoc-Phe-OH, Fmoc-Trp-OH, and Fmoc-Pro-OH.
Moreover, examples of the amino acid protected with a lipophilic protecting group having a protecting group introduced into the side chain can include Fmoc-Arg(Pbf)-OH, Fmoc-Asn(Trt)-OH, Fmoc-Asp(OtBu)-OH, Fmoc-Cys(Acm)-OH, Fmoc-Cys(StBu)-OH, Fmoc-Cys(tBu)-OH, Fmoc-Cys(Trt)-OH, Fmoc-Glu(OtBu)-OH, Fmoc-Gln(Trt)-OH, Fmoc-His(Trt)-OH, Fmoc-Lys(Boc)-OH, Fmoc-Ser(tBu)-OH, Fmoc-Thr(tBu)-OH, Fmoc-Trp(Boc)-OH, and Fmoc-Tyr(tBu)-OH.
Moreover, when it is desired to add a linker in the amino acid sequence of the sugar chain-polypeptide conjugate, a linker can be inserted at a preferred position by using a linker protected with a lipophilic protecting group instead of the above amino acid protected with a lipophilic protecting group in the solid phase synthesis process.
When employing a 2-chlorotrityl chloride resin, esterification can be carried out by employing a base such as diisopropylethylamine (DIPEA), triethylamine, pyridine, and 2,4,6-collidine. Moreover, when employing a resin possessing a hydroxyl group, for example, well-known dehydration condensing agents such as 1-mesitylenesulfonyl-3-nitro-1,2,4-triazole (MSNT), dicyclohexylcarbodiimide (DCC), and diisopropylcarbodiimide (DIC) can be employed as the esterification catalyst. The proportion of use between the amino acid and the dehydration condensing agent is 1 eq. of the former to ordinarily 1 - 10 eq., preferably 2 - 5 eq. of the latter.
The esterification reaction is preferably carried out by e.g. placing a resin in a solid phase column, washing this resin with a solvent, and then adding an amino acid solution. Examples of a washing solvent can include dimethylformamide (DMF), 2-propanol, dichloromethane, and the like. Examples of a solvent for dissolving amino acids can include dimethyl sulfoxide (DMSO), DMF, dichloromethane, and the like. The esterification reaction may be carried out at 0 - 50°C, preferably at room temperature for about 10 minutes - 30 hours, preferably 15 minutes - 24 hours.
It is also preferred at this time to cap the unreacted groups on the solid phase by acetylation with acetic anhydride etc.
The detachment of the lipophilic protecting group can be carried out by e.g. treatment with a base. Examples of a base can include piperidine, morpholine, and the like. It is preferred to do so in the presence of a solvent. Examples of a solvent can include DMSO, DMF, methanol, and the like.
The amidation reaction between the free amino group and the carboxy group of any amino acid having the amino group nitrogen protected with a lipophilic protecting group is preferably carried out in the presence of an activator and a solvent.
Examples of an activator can include dicyclohexylcarbodiimide (DCC), 1-ethyl-3-(3-dimethylaminopropyl)carbodiimide hydrochloride salt (WSC/HCl), diphenylphosphorylazide (DPPA), carbonyldiimidazole (CDI), diethylcyanophosphonate (DEPC), benzotriazol-1-yloxy-trispyrrolidinophosphonium (DIPCI), benzotriazol-1-yloxy-trispyrrolidinophosphonium hexafluorophosphate (PyBOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), 2-(1H-benzotriazol-1-yl)-1,1,3,3-tetramethyluronium hexafluorophosphate (HBTU), 1-[bis(dimethylamino)methylene]-5-chloro-1H-benzotriazolium 3-oxide hexafluorophosphate (HCTU), O-(7-azabenzotriazol-1-yl)-1,1,3,3-tetramethyluronium hexafluorophosphonate (HATU), O-benzotriazol-1-yl-1,1,3,3-tetramethyluronium tetrafluoroborate (TBTU), 3,4-dihydro-3-hydrodi-4-oxa-1,2,3-benzotriazine (Dhbt), 4-(4,6-dimethoxy-1,3,5-triazin-2-yl)-4-methylmorpholinium chloride n-hydrate (DMT-MM), and the like.

It is preferred that the amount of the activator used is 1 - 20 equivalents, preferably 1 - 10 equivalents, and further preferably 1 - 5 equivalents to the any amino acid having the amino group nitrogen protected with a lipophilic protecting group.

Examples of a solvent can include DMSO, DMF, dichloromethane, and the like. The reaction may be carried out at 0 - 50°C, preferably at room temperature for about 10 minutes - 30 hours, preferably for 15 minutes - 24 hours. The detachment of the lipophilic protecting group can be carried out similarly to the above.

When introducing a C-terminal amino acid to Rink-Amide-resin (from Merck & Co., Inc.), Rink-Amide-PEGA resin (from Merck & Co., Inc.), or NH-SAL-resin (from Watanabe Chemical Industries,Ltd.) which are functionalized with an amino group, or Amino-PEGA-resin bound to NH-SAL-resin-linker (from Merck & Co., Inc.) and the like, introduction can be carried out by employing the above amidation reaction.

Treatment with an acid is preferred for cleaving the peptide chain from the resin. Examples of an acid can include trifluoroacetic acid (TFA), hydrogen fluoride (HF), and the like.

In this way, a sugar chain-polypeptide complex possessing a glycosylated Asn at the desired position can be obtained.

In one embodiment of the present invention, when the non-reducing terminal on the sugar chain in the glycosylated Asn employed for solid phase synthesis comprises a sialic acid, it is preferred that the carboxy group of said sialic acid is protected by a protecting group in order to prevent the sialic acid from being cleaved by acid treatment. Examples of a protecting group can include a benzyl group, an allyl group, a diphenylmethyl group, a phenacyl group, and the like. The method for introducing the protecting group and detaching the protecting group can be carried out by a well-known method.

Method for manufacturing a sugar chain-polypeptide complex (Method B)

The sugar chain-polypeptide complex can also be manufactured by a method of first synthesizing a polypeptide, and then later glycosylating the synthesized polypeptide. Specifically, a polypeptide comprising Cys at the position to be glycosylated is manufactured by solid or liquid phase synthesis method, a method of synthesizing by cells, a method of separating and extracting those that occur in nature, and the like. When the polypeptide is synthesized by a solid or liquid phase synthesis method, amino acids may be linked one residue at a time, or polypeptides may be linked. Cys that is not to be glycosylated such as Cys at the position predetermined to form a disulfide bond is protected here with e.g. an acetoamidomethyl (Acm) group. Moreover, when introducing Cys that is not to be glycosylated and not used for forming a disulfide bond into the sugar chain-polypeptide complex, it can be introduced by protecting the Cys with a protecting group during the glycosylation step and the disulfide bond formation step, and then deprotecting it. Examples of such a protecting group can include tert-butyl (tBu) or 4-methoxybenzyl and the like.

Moreover, when adding different sugar chains to Cys in one polypeptide, different sugar chains can be introduced by rendering the Cys for introducing a sugar chain first unprotected, and protecting the Cys for introducing the different sugar chain next by StBu and the like. Specifically, when synthesizing the polypeptide by solid phase synthesis etc., the Cys for introducing a first sugar chain is rendered unprotected, and the Cys for introducing a second sugar chain is rendered to be a Cys possessing a protecting group with Fmoc-Cys(StBu)-OH etc. Then, a sugar chain is introduced into the unprotected Cys while the protecting group such as StBu is still retained. A different sugar chain can then be introduced into the Cys rendered unprotected by deprotecting the StBu group etc. The Cys for introducing the first sugar chain and the Cys for introducing the second sugar chain can be one or more.

The deprotection of the StBu group can be carried out by subjecting to a reaction with a reductant such as tris(2-carboxyethyl)phosphine hydrochloride salt (TCEP), dithiothreitol (DTT), and tributylphosphine. The above reaction may be carried out ordinarily at 0 - 80°C, preferably at 5 - 60°C, and further preferably at 10 - 35°C. Preferably, the reaction time is ordinarily about 30 minutes - 5 hours. Upon completion of the reaction, this may be purified with a well-known method (such as high performance liquid column chromatography (HPLC)) as appropriate.

When introducing different sugar chains, it is preferred to start the introduction with a sugar chain that is more stable against the reduction condition in the deprotection step of Cys or the acidic condition in the purification step such as HPLC. In particular, when introducing a sialic acid-containing sugar chain, it is preferred that a sugar chain that does

not possess a sialic acid or a sugar chain with less sialic acid residues is introduced first.

Moreover, when it is desired to add a linker in the amino acid sequence of the sugar chain-polypeptide complex, a linker can be inserted at a preferred position of the synthesized polypeptide by e.g. using a linker protected with a lipophilic protecting group instead of the amino acid protected with a lipophilic protecting group in the solid phase synthesis process.

Next, by reacting a haloacetylated sugar chain derivative with the peptide comprising an unprotected Cys obtained above, the sugar chain is reacted with the thiol group of the unprotected Cys and bound to the peptide. The above reaction may be carried out in a phosphate buffer, a tris-hydrochloride buffer, a citrate buffer, or a mixed solution thereof, ordinarily at 0 - 80°C, preferably at 10 - 60°C, and further preferably at 15 - 35°C. The reaction time is ordinarily 10 minutes - 24 hours, and preferably, ordinarily approximately 30 minutes - 5 hours. Upon completion of the reaction, this may be purified with a well-known method (such as HPLC) as appropriate.

An example of a haloacetylated sugar chain derivative is a compound having the hydroxyl group bound to the carbon at position 1 of an asparagine-linked sugar chain substituted with $-NH-(CH_2)_a-(CO)-CH_2X$ (wherein X is a halogen atom, and a is integer and is not limited as long as it does not inhibit the linker function of interest, preferably an integer between 0 - 4).

Specifically, the haloacetylated complex sugar chain derivative and the Cys-containing polypeptide are reacted in a phosphate buffer at room temperature. Upon completion of the reaction, the sugar chain-polypeptide complex possessing a Cys having a sugar chain bound thereto can be obtained by purification with HPLC.

The reaction can also be carried out in a mixed solution of an organic solvent such as DMSO, DMF, methanol, and acetonitrile with the above buffer. In this case, the organic solvent can be added to the above buffer at a ratio in the range of 0 - 99% (v/v) . This is preferred for a peptide comprising unprotected Cys with low solubility against the buffer because the addition of such an organic solvent can improve the solubility against the reaction solution.

The reaction can also be carried out in an organic solvent such as DMSO, DMF, methanol, and acetonitrile or a mixed solution thereof. It is preferred to do so in the presence of a base. Examples of a base can include DIPEA, triethylamine, pyridine, 2,4,6-collidine, and the like. The reaction can also be carried out in a mixed solution of guanidine hydrochloride or urea added to the buffer solution. Guanidine hydrochloride or urea can be added to the above buffer so that the final concentration will be 1 M - 8 M. This is preferred because the addition of guanidine hydrochloride or urea can also improve the solubility of a peptide with low solubility against the buffer.

Further, the reaction can also be carried out with addition of tris(2-carboxyethyl)phosphine hydrochloride salt (TCEP) or dithiothreitol (DTT) to the buffer in order to prevent the formation of a dimer of polypeptides comprising unprotected Cys via a disulfide bond. TCEP or DTT can be added to the buffer so that the final concentration will be 10 μM - 10 mM.

Moreover, when manufacturing a sugar chain-polypeptide complex possessing multiple sialic acid-containing sugar chains such as disialo or monosialo sugar chains in the peptide sequence, a sialic acid-containing sugar chain having the carboxy group of the sialic acid on the sugar chain to be introduced protected with a benzyl (Bn) group, an allyl group, a diphenylmethyl group, a phenacyl group, and the like can be employed.

When a sugar chain having the carboxy group of the sialic acid protection is introduced, a step of deprotecting the sialic acid protecting group can be carried out after a step of forming a disulfide bond in the sugar chain-polypeptide complex described below.

In this way, by protecting the carboxy group of the sialic acid with a benzyl group and the like, separation/purification step by HPLC etc. in the manufacturing step will be facilitated. Protection of the carboxy group of the sialic acid will also enable prevention of detachment of the acid-labile sialic acid.

The protection reaction of the carboxy group of the sialic acid on the sugar chain can be carried out by a method well-known to those skilled in the art. Moreover, in the sugar chain-polypeptide complex, the protecting group of the carboxy group of the sialic acid can be deprotected by hydrolysis under basic conditions. The above reaction may be carried out ordinarily at 0 - 50°C, preferably at 0 - 40°C, and further preferably at 0 - 30°C. Preferably, the reaction time is ordinarily approximately 5 minutes - 5 hours. Upon completion of the reaction, this may be purified with a well-known method (such as HPLC) as appropriate after neutralization with a weak acid such as phosphoric acid or acetic acid.

Moreover, in Method B, the amino acid to be reacted with the haloacetylated complex sugar chain derivative is not particularly limited as long as it is a thiol group-containing amino acid, and for example, D-cysteine (D-Cys), homocysteine, norcysteine, penicillamine, and the like can also be employed similarly to Cys.

The type of sugar chain bound to the sugar chain-polypeptide complex contained in the hemostatic pharmaceutical composition according to the present invention is not particularly limited, but it is preferred that the total number of sugar residues present in the sugar chain bound to the sugar chain-polypeptide complex is 5 or more. For example, one or more sugar chains that is a pentasaccharide or higher may be added, or multiple sugar chains that is a pentasaccharide or lower may be added so that the number of sugar residues that is present on the sugar chain added to one sugar chain-polypeptide complex is 5 or more. When adding multiple sugar chains, the type of sugar chain bound to one peptide may be identical or different types of sugar chains may be bound in combination, but it is preferred that they

are identical.

For example, when the total number of sugar residues present in the sugar chain bound to the sugar chain-polypeptide complex is 5, one of each of a maltose sugar chain possessing two sugar residues and a maltotriose sugar chain possessing a three sugar residues may be bound. Moreover, when the total number of sugar residues present in the sugar chain bound to the sugar chain-polypeptide complex is 6, three maltose sugar chains may be bound, or two maltotriose sugar chains may be bound. Moreover, when the total number of sugar residues present in the sugar chain bound to the sugar chain-polypeptide complex is 7, two maltose sugar chains and one maltotriose sugar chain may be bound, or one diGlcNAc sugar chain possessing seven sugar residues may be bound. Similarly, various combinations of sugar chains may be bound for cases where the total number of sugar residues present in the sugar chain bound to the sugar chain-polypeptide complex is 8 or more.

The number of sugar chains bound to the sugar chain-polypeptide complex contained in the hemostatic pharmaceutical composition according to the present invention is not limited, as long as the sugar chain-polypeptide complex will not lose the characteristic of forming a P sheet structure by self-assembly in an aqueous solution having a pH around neutral. For example, it may be 1, 2, 3, 4, 5, or 6 chains, preferably 1, 2, or 3 chains.

In the sugar chain-polypeptide complex contained in the hemostatic pharmaceutical composition according to the present invention, the position of the amino acid residue that the sugar chain binds to is not limited, as long as the sugar chain-polypeptide complex will not lose the characteristic of forming a P sheet structure by self-assembly in an aqueous solution having a pH around neutral. For example, the position of the amino acid residue that the sugar chain binds to may be the N- and/or C-terminal side of the polypeptide, or it may be a position other than the N- and C-terminal side.

Preferably, a sugar chain may be bound to every amino acid up to position x counting from the amino acid residue positioned at the N-terminal of the polypeptide and every amino acid up to position y counting from the amino acid residue positioned at the C-terminal (wherein x and y are integers, $x \geq 0$, $y \geq 0$, and $x + y$ is the total number of sugar chains bound to the polypeptide).

More specifically, when the number of sugar chains bound to the polypeptide is 1, said one sugar chain may be bound to the amino acid residue positioned at the N-terminal of said polypeptide or the amino acid residue positioned at the C-terminal.

Moreover, when the number of sugar chains bound to the polypeptide is 2, said two sugar chains may be bound to an amino acid residue selected from the group consisting of (1) - (3) below:

(1) the first and second amino acid residues counting from the amino acid residue positioned at the N-terminal of the polypeptide
(2) the first and second amino acid residues counting from the amino acid residue positioned at the C-terminal of the polypeptide, and
(3) the amino acid residue positioned at the N-terminal of the polypeptide and the amino acid residue positioned at the C-terminal of said polypeptide.

Moreover, when the number of sugar chains bound to the polypeptide is 3, said three sugar chains may be bound to any amino acid residue selected from the group consisting of (1) - (4) below:

(1) the first, second, and third amino acid residues counting from the amino acid residue positioned at the N-terminal of the polypeptide
(2) the first, second, and third amino acid residues counting from the amino acid residue positioned at the C-terminal of the polypeptide
(3) the first and second amino acid residues counting from the amino acid residue positioned at the N-terminal of the polypeptide, and the amino acid residue positioned at the C-terminal of the polypeptide, and
(4) the amino acid residue positioned at the N-terminal of the polypeptide, and the amino acid residues positioned at positions 1 and 2 counting from the C-terminal of the polypeptide.

It is preferred that the sugar chain to be added to the sugar chain-polypeptide complex contained in the hemostatic pharmaceutical composition according to the present invention is branched. Here, the sugar chain bound to the polypeptide is "a sugar chain with a branch" as used herein is not limited to e.g. those possessing a branch in one sugar chain such as with a disialo sugar chain, an asialo sugar chain, or a diGlcNAc sugar chain, but also encompasses e.g. those having multiple linear sugar chains added to one polypeptide to create a state where the sugar chain is branched in the peptide as a whole. For example, those having two or more linear sugar chains such as a maltose sugar chain or a maltotriose sugar chain bound to one peptide are also encompassed in "a sugar chain with a branch" herein.

In the present invention, the method for evaluating the strength or nature of the hydrogel is not particularly limited, and for example can be evaluated by a steel ball loading test or a kinetic viscosity measurement. In the steel

ball loading test, for example, the strength of the hydrogel can be evaluated by loading a steel ball of a given weight on the surface of a hydrogel formed inside a Durham's tube and observing whether the steel ball will stay on the surface of the hydrogel or sinks. Moreover, in the steel ball loading test, the transparency in the hydrogel or the presence or absence of an insoluble matter or precipitation can be visually verified. In the kinetic viscosity measurement of the hydrogel, the change in the strength of the hydrogel over time can be measured by measuring the kinetic viscosity of the subject hydrogel with a rheometer.

The terms used herein are to be employed to describe particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

Terms such as first and second are sometimes employed to express various elements, and it should be recognized that these elements are not to be limited by these terms. These terms are employed solely for the purpose of discriminating one element from another, and it is for example possible to describe a first element as a second element, and similarly, to describe a second element as a first element without departing from the scope of the present invention.

The present invention will now be more specifically described by Examples. However, the present invention can be embodied by various embodiments, shall not be construed as being limited to the Examples described herein.

For example, DiGlcNAc-BrAc as shown herein indicates a bromoacetylated diGlcNAc sugar chain. Moreover, for example, C(DiGlcNAc)-(RADA)4 4 as shown herein indicates that a cysteine residue having a diGlcNAc sugar chain bound thereto is bound to the N-terminal of a polypeptide having the amino acid sequence RADARADARADARADA.

## Examples

### (Synthesis Example 1) Synthesis of C(DiGlcNAc)-(RADA)4

### (Synthesis Example 1-1) Synthesis of DiGlcNAc-BrAc

Synthesis was carried out with a method similar to that described in WO2005/010053 to obtain DiGlcNAc-BrAc represented by the following Formula (12).

[Chemical Formula 12]

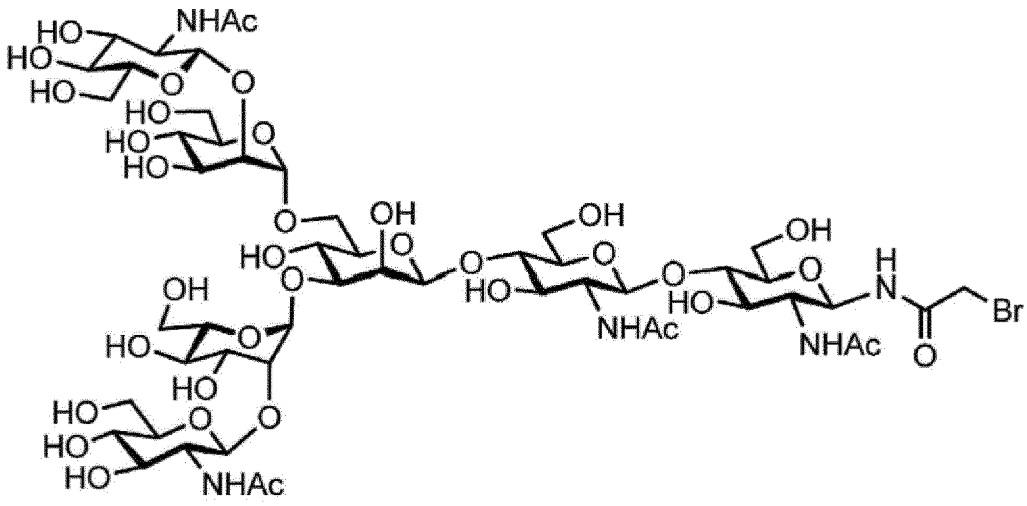

Formula (12)

(Synthesis Example 1-2) Synthesis of Ac-C(RADA)4-$NH_2$

Rink amide PEGA resin (100 μmol) was taken up in a column for solid phase synthesis, washed with DMF and dichloromethane, followed by addition of a solution of Fmoc-Ala-OH (124.5 mg, 400 μmol), 1-bisdimethylaminomethylen-5-chloro-1H-benzotriazolium 3-oxide hexafluorophosphate (HCTU) (157.2 mg, 380 μmol), and diisopropylethylamine (DIPEA) (104.5 μL, 600 μmol) in DMF (2.5 mL), and this was shaken for 15 minutes. After washing with dichloromethane and DMF, the Fmoc protecting group was removed by treatment with 20% piperidine in DMF. After washing with DMF, a resin-bound polypeptide protected with peptide solid phase synthesis method by Fmoc method represented by the following Formula (13) (SEQ ID NO. 3) was synthesized with a Prelude™ peptide synthesizer. The condensation reaction was carried out in DMF using HCTU as the condensing agent.

[Chemical Formula 13]

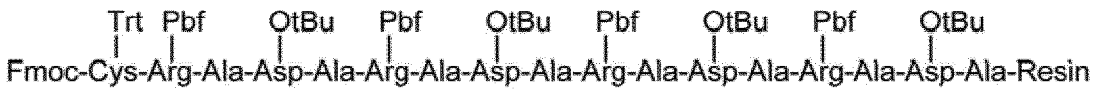

Formula (13)

The Fmoc protecting group was removed by treatment with 20% piperidine in DMF. After washing with DMF and dichloromethane, acetic anhydride and pyridine were added and shaken for 1 hour. After washing with DMF and dichloromethane, trifluoroacetic acid (TFA) :water:triisopropylsilane:ethanedithiol (= 90:2.5:5:2.5) was added, and this was shaken for 4 hours at room temperature. The resin was filtered off, chilled diethyl ether was added to the filtrate, and crude peptide was obtained as the precipitate. A portion of the crude peptide was purified with HPLC [column: SHISEIDO CAPCELL PAK C18 UG-120 (5 μm), φ 20 x 250 mm, flow rate: 7.0 mL/min, developing solvent A: 0.1% aq. TFA, B: 0.09% TFA/10% water/90% acetonitrile, gradient A:B = 88:12 -> 78:22, 11 min. linear concentration gradient elution] to obtain a polypeptide represented by the following Formula (14) (SEQ ID NO. 4) (32.7 mg).
[Chemical Formula 14]

**Ac-Cys-Arg-Ala-Asp-Ala-Arg-Ala-Asp-Ala-Arg-Ala-Asp-Ala-Arg-Ala-Asp-Ala-$NH_2$** Formula (14)

(Synthesis Example 1-3) Synthesis of C(DiGlcNAc)-(RADA)4 4

The polypeptide synthesized in Synthesis Example 1-2 (SEQ ID NO. 4) (25.3 mg, 13.9 μmol) and DiGlcNAc-BrAc synthesized in Synthesis Example 1-1 (30.0 mg, 20.9 μmol, 1.5 eq. to peptide 1) were dissolved in 0.2 M phosphate buffer (pH 7.3, 4.7 mL) comprising 33 μM TCEP and 8 M guanidine hydrochloride, and reacted at room temperature for 3 hours.

The reaction solution was purified with HPLC [column: SHISEIDO CAPCELL PAK C18 UG-120 (5 μm), φ 20 x 250 mm, flow rate: 7.0 mL/min, developing solvent A: 0.1% aq. TFA, B: 0.09% TFA/10% water/90% acetonitrile, gradient A:B = 88:12 -> 81:19, 10 min. linear concentration gradient elution] to obtain a sugar chain-polypeptide complex represented by the following Formula (15) (SEQ ID NO. 5) (23.9 mg, 7.53 μmol, yield 54%).

[Chemical Formula 15]

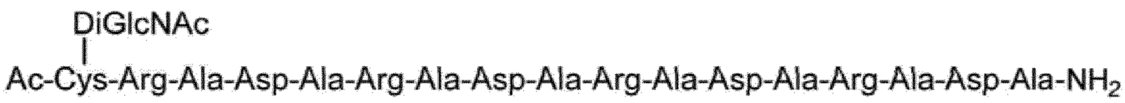

Formula (15)

(Example 1) Measurement and analysis of circular dichroism (CD)

Self-assembling peptides such as (RADA)4 4 are known to form a β sheet structure due to intermolecular interaction, and further this structure is layered in many layers in the presence of ions to form a hydrogel. CD measurement is known as an effective means to confirm this sheet structure. In general, the CD spectrum when a β sheet structure is present shows a positive maximum at around 197 nm and a negative maximum at around 216 nm. Accordingly, it

was confirmed whether the composition of the present invention forms a β sheet structure in a broad pH by carrying out CD measurement.

C(DiGlcNAc)-(RADA)4 synthesized in Synthesis Example 1 and (RADA)4 (product name: PuraMatrix from 3D Matrix, Product No:354250) as the control were each dissolved in ultrapure water to prepare 1% by weight aqueous solutions. To these aqueous solutions were added equal amounts of ultrapure water, 1.8% saline, or 0.3 M phosphate buffer (pH 7.4) to produce an aqueous solution and a hydrogel at 0.5% by weight, respectively. These were then diluted with ultrapure water so that the peptide concentration of each solution was 100 mM. These solutions were transferred to quartz cells having optical path length of 0.1 cm. The CD spectrum was then measured with a circular dichroism spectrometer (J-805, Jasco) at wavelengths of 190 - 260 nm. The mean residue ellipticity) θ was calculated with the following formula:

$$[\theta] = (\theta_{obs}/10 \cdot 1 \cdot c)/r$$

wherein $\theta_{obs}$ represents the ellipticity measured in millidegree, 1 represents the cell length (cm), c represents the concentration (M), and r represents the number of amino acid residues.

The measurement result for a composition comprising C(DiGlcNAc)-(RADA)4 is shown in Figure 1, and the measurement result for a composition comprising (RADA)4 is shown in Figure 2.

In an aqueous solution and physiological saline, both C(DiGlcNAc)-(RADA) 4 and (RADA)4 showed a positive maximum at around 197 nm and a negative maximum at around 216 nm, thus confirming that both peptides formed β sheet structures. On the other hand, in a phosphate buffer, formation of a β sheet structure was confirmed only for C(DiGlcNAc)-(RADA)4.

<u>(Example 2) Confirmation of formation of fibrous structure</u>

C(DiGlcNAc)-(RADA)4 synthesized in Synthesis Example 1 and (RADA)4 as the control were each dissolved in ultrapure water to prepare 1% by weight aqueous solutions. Each of the aqueous solutions prepared were each diluted with ultrapure water, 1.8% saline, or phosphate buffer (pH 7.4) to obtain 0.5% by weight. One micro liter of the diluted solution was each added dropwise onto a cleaved mica substrate (product name: MICA Grade V-4 from SPI supplies). Excess compound on said mica substrate was then washed away with 100 μL of distilled water. The substrate was then air dried at room temperature (25°C).

The peptide on the mica substrate after drying was observed with an atomic force microscope (product name: Nanoscale Hybrid Microscope VN-8000 from Keyence). The results are shown in Figure 1.

As shown in Figure 3, it was found that C (DiGlcNAc)-(RADA)4 self-assembles and forms a fibrous structure under any of the conditions. On the other hand, for (RADA)4, it was found that what looked like aggregates were observed in phosphate buffer and a fibrous structure was not formed.

<u>(Example 3) Measurement and analysis of kinetic viscosity</u>

A rheometer (Discovery HR-2 from TA Instruments) equipped with stainless steel parallel plates with a gap height of 0.3 mm and 40 mm diameter was employed for measurement of kinetic viscosity. C(DiGlcNAc)-(RADA)4 was dissolved in ultrapure water to prepare 0.5 - 5% by weight aqueous peptide solutions. (RADA)4 as the control was also dissolved in ultrapure water to prepare 0.5 - 1% by weight aqueous peptide solutions. These aqueous solutions were then transferred to a rheometer set at 25°C, Preshear was applied for 30 seconds at a rotation speed of 100 $S^{-1}$, after which various physical property data were monitored over time (frequency = 1Hz, distortion = 10%). This measurement result is shown in Figure 4.

After dissolving C(DiGlcNAc)-(RADA)4 in ultrapure water, an equal amount of phosphate buffer (300 mM, pH 7.4) was added to produce a hydrogel at 0.5 - 5% by weight. In addition, after dissolving (RADA)4 in ultrapure water, an equal amount of 1.8% saline was added to produce a hydrogel at 0.5 - 1% by weight. Note that (RADA)4 did not completely dissolve at a concentration of 2% by weight or higher, making handling difficult and thus the test could not be carried out. The kinetic viscosity of these hydrogels was measured under conditions similar to the above. This measurement result is shown in Figure 5.

Figure 4 shows the storage elastic modulus in aqueous solution state of C(DiGlcNAc)-(RADA)4 and (RADA)4. Comparing the storage elastic modulus in aqueous solution state of C(DiGlcNAc)-(RADA)4 at 1% by weight and (RADA)4 at 1% by weight, C(DiGlcNAc)-(RADA)4 showed a lower value. On the other hand, similarly comparing the storage elastic modulus of each in hydrogel state after addition of salt as shown in Figure 5, C(DiGlcNAc)-(RADA)4 and (RADA)4 showed equivalent values. From these results, it was found that C(DiGlcNAc)-(RADA)4 has a characteristic of having

low storage elastic modulus in aqueous solution state and is easy to handle, while gelling instantly when salt is added. Moreover, because C(DiGlcNAc)-(RADA)4 has high solubility in water, aggregates will not be produced even when the peptide concentration is increased, and a hydrogel having high storage elastic modulus can be provided.

(Example 4) Evaluation of hemostatic action

In order to confirm whether the hydrogel of the present invention has hemostatic action *in vivo,* an evaluation test in a hemorrhage model by liver puncture of a rat was carried out.

A 9-week-old Crlj:SD rat was anesthetized with isoflurane, and heparin sodium injection (200 U/rat from Ajinomoto Pharmaceuticals Co., Ltd.) was administered from the tail vein. The rat was subjected to abdominal section, and a plastic paraffin film was placed under the lateral left lobe and medial right lobe of the liver to expose the liver surface. The same site of the hepatic lobe was punctured three times with a needle apparatus (pins were bundled to about 7 mm diameter in order to make puncture to a depth of 3 - 4 mm) to give a liver puncture model. Bleeding up to 10 seconds after puncture was removed with an absorbent cotton, and then 100 μL each of the test substance (C(DiGlcNAc)-(RADA)4, (RADA)4) and vehicle (purified water, PBS) were immediately locally administered dropwise to the punctured site. At 1.5 and 3 minutes after administration, hemostatic and hemorrhage state was verified by the scoring criteria below to evaluate the hemostatic effect of the test substance. C(DiGlcNAc)-(RADA)4 was dissolved in PBS (pH 7.4, phosphate buffered saline) to 0.5% by weight and used for evaluation. (RADA)4 was dissolved in purified water to 0.5% by weight and used for evaluation. [Table 1]

Table 1 Verification score for hemostatic and hemorrhage state

| Assessment | Score | State of punctured site |
|---|---|---|
| O:Hemostatic | 2 | State where hemorrhage is not observed. |
| Δ:Mostly hemostatic | 1 | State where slight hemorrhage is seen even if only intermittent. |
| ×:Hemorrhage | 0 | State where hemorrhage is intermittently seen. |

The evaluation result of hemostatic effect is shown in Table 2. In addition, score distribution of the hemostatic effect after 3 minutes is shown in Figure 6. The number of individuals where continuous hemorrhage was observed until after 3 minutes were 12 out of 16 cases (75%) for the PBS application group and 13 out of 15 cases (87%) for the purified water application group. On the other hand, the number of individuals where continuous hemorrhage was observed until after 3 minutes for the C(DiGlcNAc)-(RADA)4 application group was 4 out of 15 cases (27%), and the number of individuals where continuous hemorrhage was observed until after 3 minutes for the (RADA)4 application group was 3 out of 15 cases (20%) . The remaining individuals formed a gel-like solid or a coating film and the like by 3 minutes after puncture, and the flow of blood from the hemorrhage site had stopped or the amount of flow decreased.

Because in this test system heparin is administered to the rat before administering the test substance to make the examination under conditions where the intrinsic blood coagulation system is suppressed, the above result shows the hemostatic effect that the test substance possesses. [Table 2]

Table 2

| | Treatment | C(DiGlcNAc)-(RADA) 4 | PBS | (RADA) 4 | Purified water |
|---|---|---|---|---|---|
| 1.5 minutes | Continuous hemorrhage (score 0) | 9/15 (60%) | 16/16 (100%) | 11/15 (73%) | 14/15 (93%) |
| | Mostly hemostatic (score 1) | 4/15 (27%) | 0/16 (0%) | 3/15 (20%) | 0/15 (0%) |
| | Hemostatic (score 2) | 2/15 (13%) | 0/16 (0%) | 1/15 (7%) | 1/15 (7%) |
| 3 minutes | Continuous hemorrhage (score 0) | 4/15 (27%) | 12/16 (75%) | 3/15 (20%) | 13/15 (87%) |
| | Mostly hemostatic (score 1) | 1/15 (7%) | 1/16 (6%) | 2/15 (13%) | 0/15 (0%) |
| | Hemostatic (score 2) | 10/15 (67%) | 3/16 (19%) | 10/15 (67%) | 2/15 (13%) |

From the above results, it was found that (C(DiGlcNAc)-(RADA)4 has higher usability compared to (RADA)4 while showing a hemostatic effect equivalent to that of (RADA) 4. In other words, it was shown that the hydrogel of the present invention has extremely high utility value as a hemostatic pharmaceutical composition.

## Claims

1. A hemostatic pharmaceutical composition comprising a sugar chain-polypeptide complex **characterized in that** said polypeptide in said sugar chain-polypeptide complex is a polypeptide comprising an amino acid sequence in which polar and nonpolar amino acid residues are alternately arranged, and
one or more sugar chains are bound to said polypeptide.

**2.** A hemostatic pharmaceutical composition according to claim 1, **characterized in that** said polypeptide in said sugar chain-polypeptide complex is a polypeptide comprising an amino acid sequence consisting of 8 - 34 amino acid residues in which polar and nonpolar amino acid residues are alternately arranged.

**3.** A hemostatic pharmaceutical composition according to claim 1 or 2, **characterized in that** said sugar chain-polypeptide complex may form a hydrogel comprising a β sheet structure by self-assembly in an aqueous solution having a pH around neutral.

**4.** A hemostatic pharmaceutical composition according to claim 1, **characterized in that** the concentration of said sugar chain-polypeptide complex contained in said hemostatic pharmaceutical composition is 0.1% by weight - 20% by weight.

**5.** A hemostatic pharmaceutical composition according to claim 1, **characterized in that** the total number of sugar chain residues present in the one or more sugar chains bound to said polypeptide is 5 or more.

**6.** A hemostatic pharmaceutical composition according to claim 5, **characterized in that** the number of sugar chains bound to said polypeptide is 1, 2, or 3.

**7.** A hemostatic pharmaceutical composition according to claim 5, **characterized in that** sugar chains are bound to every amino acid up to position x counting from the amino acid residue positioned at the N-terminal of said polypeptide and every amino acid up to position y counting from the amino acid residue positioned at the C-terminal (wherein x and y are integers, $x \geq 0$, $y \geq 0$, and $x + y$ is the total number of sugar chains bound to the polypeptide).

**8.** A hemostatic pharmaceutical composition according to claim 1, **characterized in that** said sugar chain is a sugar chain with a branch.

**9.** A hemostatic pharmaceutical composition according to any one of claims 1 to 8, **characterized in that** said pharmaceutical composition is in a hydrogel state.

[Figure 1]

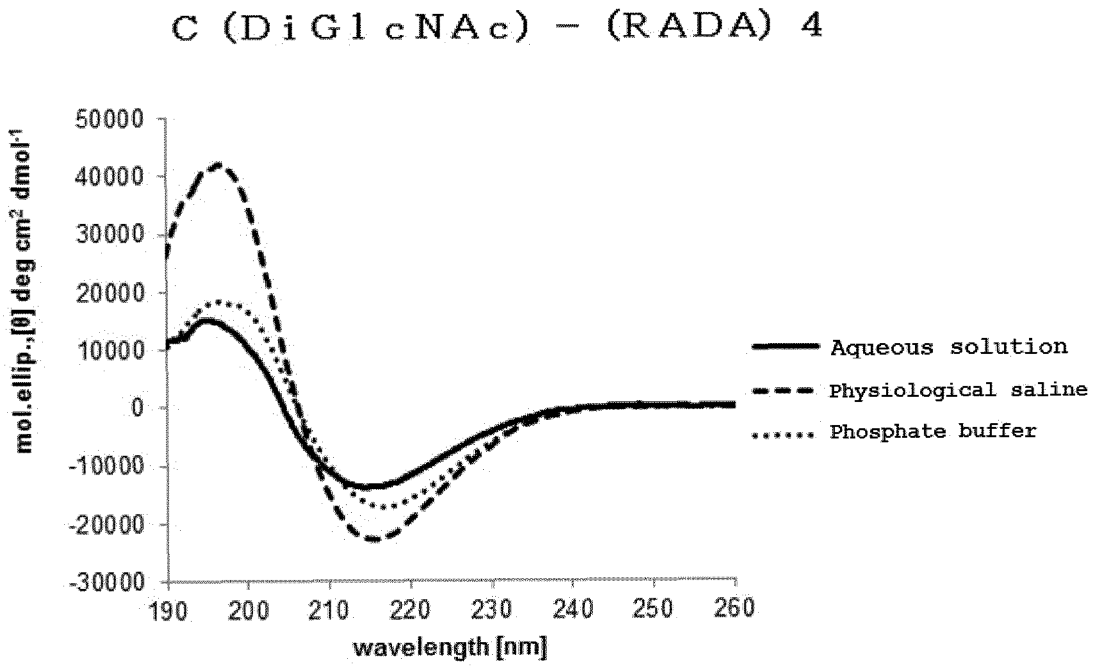


[Figure 2]

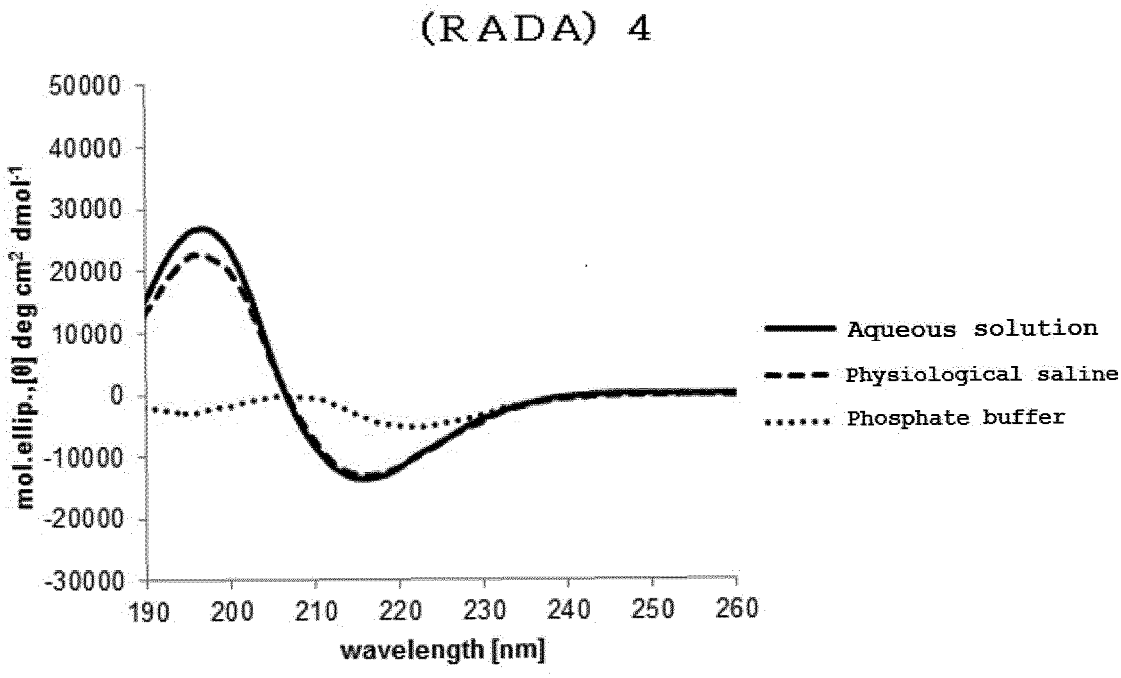

[Figure 3]

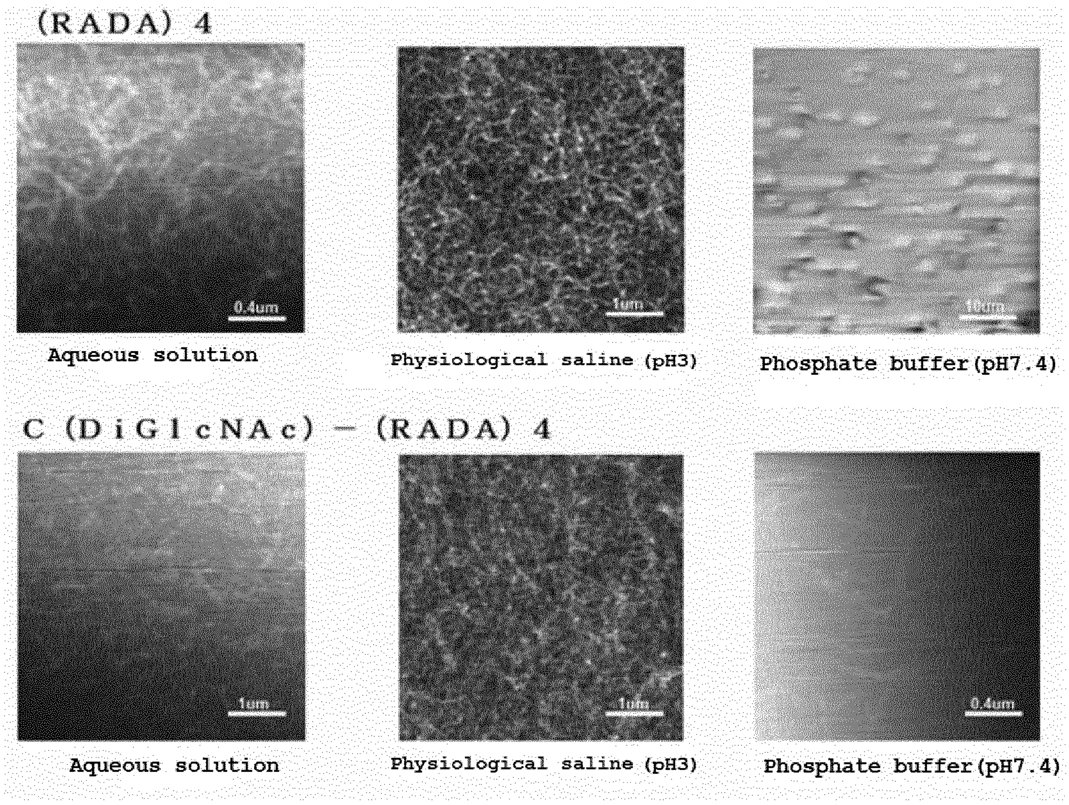


[Figure 4]

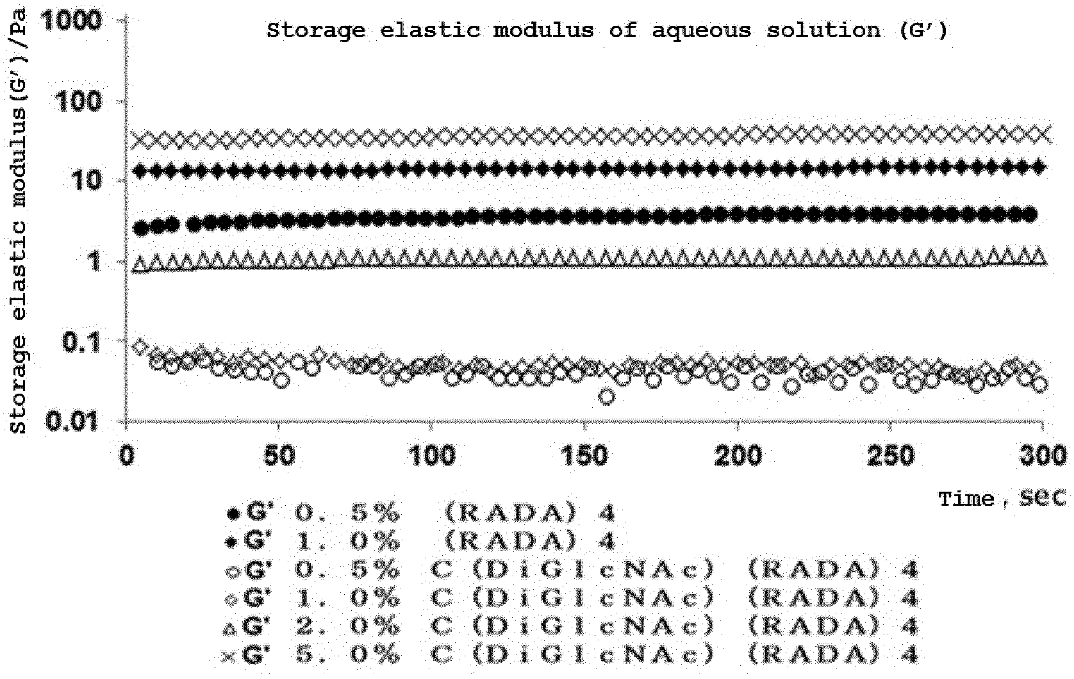

[Figure 5]

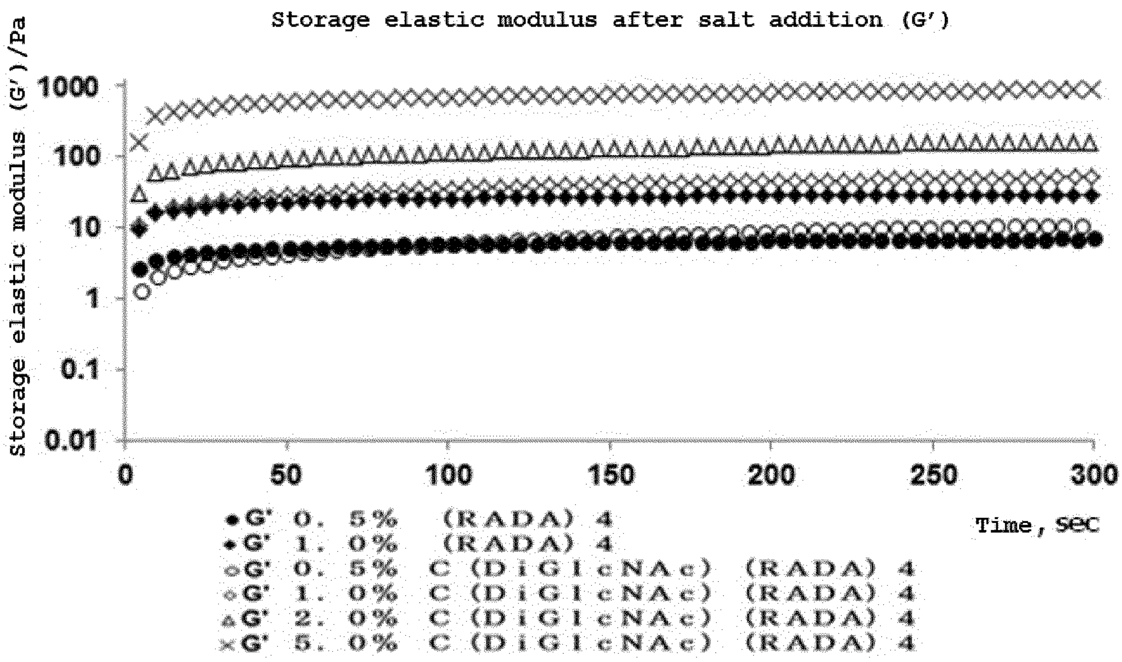


[Figure 6]

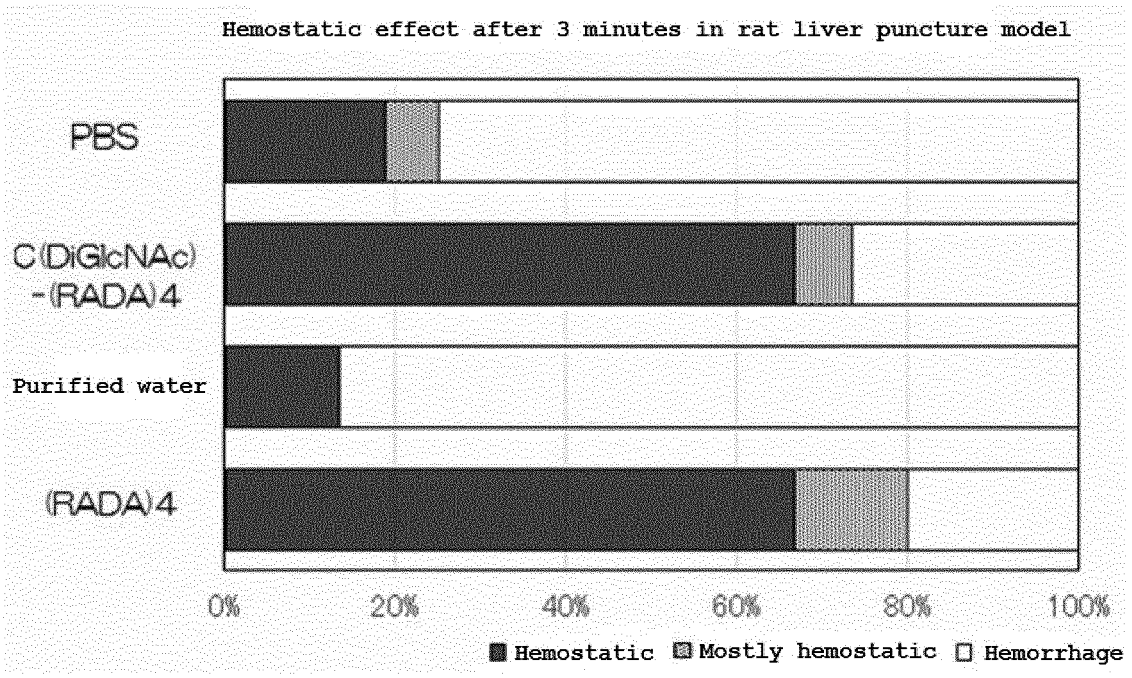

**INTERNATIONAL SEARCH REPORT**

International application No. PCT/JP2014/070703

A. CLASSIFICATION OF SUBJECT MATTER
*A61L31/00*(2006.01)i, *C07K9/00*(2006.01)n

According to International Patent Classification (IPC) or to both national classification and IPC

B. FIELDS SEARCHED

Minimum documentation searched (classification system followed by classification symbols)
A61L31/00, C07K9/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2007-217376 A (Nagoya Institute of Technology), 30 August 2007 (30.08.2007), claims; paragraphs [0008] to [0046], [0065] to [0069]; examples (Family: none) | 1-9 |
| A | WO 2010/41636 A1 (3-D Matrix, Ltd.), 15 April 2010 (15.04.2010), entire text & US 2011/0201541 A1 & EP 2345433 A1 | 1-9 |
| A | JP 2010-521495 A (Arch Therapeutics, Inc.), 24 June 2010 (24.06.2010), entire text & US 2008/0274979 A1 & EP 2146733 A & WO 2008/113030 A2 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 October, 2014 (01.10.14) | 14 October, 2014 (14.10.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/070703

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-539257 A (Massachusetts Institute of Technology), 13 November 2008 (13.11.2008), entire text & US 2007/0203062 A1 & EP 1879606 A & WO 2006/116524 A1 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5670483 A
- WO 2004005330 A
- US 2005222382 A1
- WO 2005010053 A
- US 2007060543 A1
- WO 03008431 A
- US 2004181054 A1
- WO 2004058984 A
- US 2006228784 A1
- WO 2004058824 A
- US 2006009421 A1
- WO 2004070046 A
- US 2006205039 A1
- WO 2007011055 A

SEQUENCE LISTING

<110>  GLYTECH, INC.

<120>  PHARMACEUTICAL COMPOSITION FOR BLOOD STANCHING

<130>  OKKP1401F

<150>  JP2014-068333
<151>  2014-03-28

<160>  5

<170>  PatentIn version 3.5

<210>  1
<211>  16
<212>  PRT
<213>  artificial sequence

<220>
<223>  chemically synthesized

<400>  1

```
Arg Ala Asp Ala Arg Ala Asp Ala Arg Ala Asp Ala Arg Ala Asp Ala
1               5                   10                  15
```

<210>  2
<211>  20
<212>  PRT
<213>  artificial sequence

<220>
<223>  chemically synthesized

<400>  2

```
Arg Ala Asp Ala Arg Ala Asp Ala Arg Ala Asp Ala Arg Ala Asp Ala
1               5                   10                  15


Arg Ala Asp Ala
            20
```

<210>  3
<211>  17
<212>  PRT
<213>  artificial sequence

<220>
<223>  chemically synthesized

<220>
<221>  BINDING
<222>  (1)..(1)
<223>  Fmoc

<220>
<221>  BINDING

<222> (1)..(1)
<223> Trt

<220>
<221> BINDING
<222> (2)..(2)
<223> Pbf

<220>
<221> BINDING
<222> (4)..(4)
<223> OtBu

<220>
<221> BINDING
<222> (6)..(6)
<223> Pbf

<220>
<221> BINDING
<222> (8)..(8)
<223> OtBu

<220>
<221> BINDING
<222> (10)..(10)
<223> Pbf

<220>
<221> BINDING
<222> (12)..(12)
<223> OtBu

<220>
<221> BINDING
<222> (14)..(14)
<223> Pbf

<220>
<221> BINDING
<222> (16)..(16)
<223> OtBu

<220>
<221> BINDING
<222> (17)..(17)
<223> Resin

<400> 3

```
Cys Arg Ala Asp Ala Arg Ala Asp Ala Arg Ala Asp Ala Arg Ala Asp
1               5                   10                  15


Ala
```

<210> 4
<211> 17
<212> PRT
<213> artificial sequence

```
<220>
<223>  chemically synthesized


<220>
<221>  ACETYLATION
<222>  (1)..(1)

<220>
<221>  AMIDATION
<222>  (17)..(17)

<400>  4

Cys Arg Ala Asp Ala Arg Ala Asp Ala Arg Ala Asp Ala Arg Ala Asp
1               5                   10                  15


Ala



<210>  5
<211>  17
<212>  PRT
<213>  artificial sequence

<220>
<223>  chemically synthesized


<220>
<221>  ACETYLATION
<222>  (1)..(1)

<220>
<221>  CARBOHYD
<222>  (1)..(1)
<223>  DiGlcNAc sugar chain added

<220>
<221>  AMIDATION
<222>  (17)..(17)

<400>  5

Cys Arg Ala Asp Ala Arg Ala Asp Ala Arg Ala Asp Ala Arg Ala Asp
1               5                   10                  15


Ala
```